# EUROPEAN PATENT SPECIFICATION

(11) **EP 2 752 275 B1**
(45) Date of publication and mention of the grant of the patent: **23.03.2016**
(21) Application number: 11871744.6
(22) Date of filing: 02.09.2011
(51) Int. Cl.: B26B 1/08, A61B 17/3211, B26B 21/40

(54) **CUTTING INSTRUMENT**
SCHNEIDINSTRUMENT
INSTRUMENT TRANCHANT

(43) Date of publication of application: 09.07.2014
(73) Proprietor: Feather Safety Razor Co., Ltd., Osaka-shi, Osaka 531-0075 (JP)
(72) Inventor: ICHIYANAGI Masao, Seki-shi Gifu 501-3881 (JP); TAKAHASHI Akira, Nagoya-shi Aichi 453-0018 (JP)
(74) Representative: Kramer Barske Schmidtchen Patentanwälte PartG mbB
(86) International application number: PCT/JP2011/070029
(87) International publication number: WO 2013/031017

(56) References cited:
- WO-A1-2008/029566
- JP-A- H06 170 064
- JP-A- 2007 061 429
- JP-A- 2007 159 938
- JP-U- H0 267 962
- US-A- 1 506 897
- US-A- 1 888 488
- US-A- 3 262 205
- US-A- 4 660 287

## Description

### TECHNICAL FIELD

The present invention relates to a cutter configured by detachably attaching a replaceable blade to a cutter body.

### BACKGROUND ART

As a cutter used for a knife for medical use, a knife for pathologic use, or razors for facial shaving and haircut, there is a cutter configured by detachably attaching a replaceable blade to a cutter body.

In such a cutter, the replaceable blade is attached to the cutter body by inserting the replaceable blade into a groove section provided in the cutter body. Then, the replaceable blade can be replaced after a predetermined number of use or a period of use.

Various devising is made to such a blade-replaceable type cutter in order to facilitate an easy replacement of the replaceable blade. For example, a cutter described in a patent document 1 includes an engaging member capable of sliding with respect to a cutter body, and the replaceable blade can be attached to and detached from the cutter body by using a fore end section of the cutter body and the engaging member such that an opening section provided in the replaceable blade is engaged and being pulled in a longitudinal direction.

Further, a patent document 2 discloses the cutter that allows a replaceable blade cartridge configured of a replaceable blade and a protection cover to be detachably attached to a cutter body.

### PRIOR ART DOCUMENT

### Patent Document

Patent Document 1: JP-A-200_7-061429
Patent Document 2: US 7,207,999 B2

The two part form of claim 1 is based on Patent Document 1.

### SUMMARY OF THE INVENTION

### PROBLEM TO BE SOLVED BY THE INVENTION

However, in the cutter of the patent document 1, a coil spring is used for retaining the engaging member at a rear end side in the longitudinal direction so that the replaceable blade does not fall off. That is, the replaceable blade is retained on the cutter body by the engaging member which is pulled to the rear end side in the longitudinal direction by a biasing force of the coil spring. Due to this, the coil spring needs to be installed in the cutter body, which causes problems that a configuration thereof becomes complicated, and a number of components increase.

Further, the cutter of the patent document 2 is capable of replacing the replaceable blade cartridge configured of the replaceable blade and the protection cover, as described above. That is, unlike the replaceable blade as the disposable component, the replaceable blade cartridge configured of the replaceable blade and the protection cover will be a disposable component. A long-term use of the cutter of the aforementioned blade-replaceable type may increase the cost (running cost), which is not economical.

The present invention has been made in view of the foregoing problems, and aims to provide a cutter which allows replacement of the replaceable blade only, and has less number of components, and a simple configuration.

### MEANS FOR SOLVING THE PROBLEM

An aspect of the present invention is a cutter configured by detachably attaching a replaceable blade to a cutter body. The cutter body is configured of a main body block and a slide block retained with respect to the main body block so as to be able to move in a longitudinal direction of the 15 cutter. The main body block includes a container section that houses the slide block so as to allow the slide block to move in the longitudinal direction, a main body supporting section that supports a back face of the replaceable blade, and a main body connecting section that connects with the replaceable 20 blade from a front face side. The slide block includes a rear-side supporting section that supports the back face of the replaceable blade at a rear side of the main body supporting section, a slide connecting section that connects with the replaceable blade so as to retract the replaceable blade toward 25 the rear side, a flexible section that bends in a thickness direction of the replaceable blade at the rear side of the rear-side supporting section, and a wing section that protrudes from a part of the flexible section toward at least one side in a height direction perpendicular to the longitudinal direction and the thickness direction. A front side retaining section that retains the wing section from a front side is formed in at least one side of the container section in the height direction, and the front side retaining section includes a protruding engaging section that protrudes toward a backside. In an attached state in which the replaceable blade is attached to the cutter body, the wing section is engaged from a foreside by the protruding engaging section, and the wing section is configured capable of being released from the protruding engaging section by bending the flexible section of the slide block from the attached state. Preferred embodiments are disclosed in the dependent claims.

### EFFECTS OF THE INVENTION

In the cutter as described above, when the replaceable blade is to be attached to the cutter main body, a blade replacing state in which the replaceable blade can be detachably attached is realized by moving the slide block forward, and the replaceable blade is temporarily connected with the main body connecting section and the slide connecting section. Then the main body connecting section and the slide connecting section pull the replaceable blade to both sides in the longitudinal direction by causing the slide block to retract rearward. Due to this, the attached state in which the replaceable blade is attached to the cutter body is realized.

Further, contrary to the above, the replacement of the replaceable blade may be easily performed by forcibly moving the slide block forward from the attached state to realize the blade replacing state.

Further, in the attached state, the wing section is engaged from the foreside by the protruding engaging section. Due to this, the slide block is restricted to move forward, and the attached state is maintained. Further, the wing section is configured to be releasable from the protruding engaging section by bending the flexible section of the slide block in the attached state.

That is, in the cutter, fixation of the slide block in the attached state and switching from the attached state to the blade replacing state can both easily be implemented by utilizing the elastic deformation of the flexible section of the slide block. Thus, no additional member needs to be provided for restricting the slide block to move forward in the attached state, and the configuration for releasing the attached state can be simplified to a great extent.

This allows the cutter to reduce the number of components and have a simplified configuration.

As described above, the present invention provides the cutter which allows replacement of only the replaceable blade, reduced number of components, and simplified configuration.

### BRIEF DESCRIPTION OF THE DRAWINGS

FIG. 1 is a front view of a replaceable blade and a cutter body according to a first embodiment.
FIG. 2 is a front view of a cutter according to the first embodiment.
FIG. 3 is a perspective view of the cutter according to the first embodiment.
FIG. 4 is a bottom view of the cutter according to the first embodiment.
FIG. 5 is a backside view of the cutter according to the first embodiment.
FIG. 6 is a partial cross sectional view of the cutter according to the first embodiment.
FIG. 7 is an enlarged cross sectional view along a line A-A according to FIG. 2.
FIG. 8 is an enlarged cross sectional view of a periphery of a wing section in an attached state and a blade replacing state according to the first embodiment.
FIG. 9 is an enlarged explanatory cross sectional view of the periphery of the wing section in the blade replacing state according to the first embodiment.
FIG. 10 is a cross sectional view along a line B-B of FIG. 2.
FIG. 11 is a front view of the cutter body according to the first embodiment.
FIG. 12 is a bottom view of the cutter body according to the first embodiment.
FIG. 13 is a perspective view of the cutter body according to the first embodiment.
FIG. 14 is a front view of a main body block according to the first embodiment.
FIG. 15 is a bottom view of the main body block according to the first embodiment.
FIG. 16 is a backside view of the main body block according to the first embodiment.
FIG. 17 is a perspective view of the main body block according to the first embodiment.
FIG. 18 is a cross sectional view along a line C-C of FIG. 14.
FIG. 19 is a front view of a slide block according to the first embodiment.
FIG. 20 is a bottom view of the slide block according to the first embodiment.
FIG. 21 is a perspective view of the slide block according to the first embodiment.
FIG. 22 is a front view of the replaceable blade according to the first embodiment.
FIG. 23 is a cross sectional view along a line D-D of FIG. 2.
FIG. 24 is a front view of a fore end portion of the cutter according to the first embodiment.
FIG. 25 is a cross sectional view along a line E-E of FIG. 24.
FIG. 26 is a front view of the fore end portion of the cutter in the blade replacing state according to the first embodiment.
FIG. 27 is a cross sectional view along a line F-F of FIG. 26.
FIG. 28 is a front view of the cutter body in the blade replacing state according to the first embodiment.
FIG. 29 is a perspective view of a cutter having a slide cover arranged at a foreside position according to a second embodiment.
FIG. 30 is a front view of the cutter having the slide cover arranged at the foreside position according to the second embodiment.
FIG. 31 is a backside view of the cutter having the slide cover arranged at the foreside position according to the second embodiment.
FIG. 32 is a perspective view of the cutter having the slide cover arranged at a backside position according to the second embodiment.
FIG. 33 is another perspective view of the cutter having the slide cover arranged at the backside position according to the second embodiment.
FIG. 34 is a front view of the cutter having the slide cover arranged at the backside position according to the second embodiment.
FIG. 35 is a bottom view of the cutter having the slide cover arranged at the backside position according to the second embodiment.
FIG. 36 is a backside view of the cutter having the slide cover arranged at the backside position according to the second embodiment.
FIG. 37 is a perspective view of the slide cover according to the second embodiment.
FIG. 38 is another perspective view of the slide cover according to the second embodiment.
FIG. 39 is a backside view of the slide cover according to the second embodiment.
FIG. 40 is a cross sectional view along a line G-G of FIG. 37.
FIG. 41 is a partial backside view of a main body block (cutter body) according to the second embodiment.
FIG. 42 is a bottom view of a cutter according to a third embodiment.
FIG. 43 is a front view of the cutter according to a third embodiment.
FIG. 44 is a bottom view of a cutter body in a blade replacing state according to a third embodiment.
FIG. 45 is a front view of the cutter body in the blade replacing state according to a third embodiment.
FIG. 46 is a perspective view of a main body block 4 according to a third embodiment.
FIG. 47 is an enlarged explanatory diagram in the vicinity of a lower side opening according to a third embodiment.
FIG. 48 is a front view of a slide block according to a third embodiment.
FIG. 49 is a bottom view of the slide block according to a third embodiment.
FIG. 50 is a perspective view of the slide block according to a third embodiment.

### MODE TO CARRYING OUT THE INVENTION

The cutter of the present invention may for example be a knife for medical use, a knife for pathologic use, or razors for facial shaving and haircut.

Further preferably, the protruding engaging section includes a tapered section at a fore end section, and a protruding amount of the tapered section decreases toward a fore end side. In this case, when sliding the slide block from the blade replacing state to the attached state, the wing section can be moved to a back face of the protruding engaging section along the tapered section. Further, by causing the slide block to slide toward a rear-side as it is, the wing section can easily be engaged with a rear end section of the protruding engaging section. Due to this, the movement of the slide block from the blade replacing state to the attached state can smoothly be performed.

Preferably, the replaceable blade has flexibility in the thickness direction, and elastically deforms in the attached state so as to curve in the thickness direction protrudingly toward a backside. In this case, the replaceable blade is to be pressed against the main body supporting section, the main body connecting section, and the rear-side supporting section by an elastic force (restoring force) of itself. Due to this, the replaceable blade can more stably be fixed to the cutter body.

Preferably, in the attached state, the rear-side supporting section protrudes toward a front side in the thickness direction farther than the main body supporting section. In this case, the replaceable cutter that is engaged with the main body connecting section and is supported at its back face by the main body supporting section and the rear-side supporting section can easily curve in the protruding shape toward the main body supporting section and the rear-side supporting section. Due to this, the replaceable blade can surely be fixed to the cutter body by the elastic force of the replaceable blade.

Further, the replaceable blade preferably includes an opening section that penetrates the replaceable blade in the thickness direction, the main body connecting section protrudes to the front side in the thickness direction from the main body supporting section, and is inserted in the opening section and abuts on a fore edge of the opening section in the attached state, and the slide connecting section protrudes to the front side in the thickness direction from the rear-side supporting section, and is inserted in the opening section and abuts on a rear end of the opening section in the attached state. In this case, the replaceable blade can easily and surely be connected by the main body connecting section and the slide connecting section, and the attached state can easily be established.

Further, in this case, the replaceable blade can be attached to the cutter body as below, for example. Firstly, the slide block is moved forward to realize the blade replacing state, and the main body connecting section and the slide connecting section are inserted into the opening section of the, replaceable blade. Then, the slide block is moved rearward, whereby the main body connecting section is caused to abut on the fore edge of the opening section of the replaceable blade, and the slide connecting section is caused to abut on the rear end of the opening section of the replaceable blade. Due to this, the attached state in which the replaceable blade is attached to the cutter body can easily be formed.

Preferably, the slide block includes a rear-side opposing face that opposes a rear end of the replaceable blade in the longitudinal direction. The backside opposing face is configured to press the replaceable blade toward the foreside by the backside opposing face abutting on the back end of the replaceable blade when the slide block is moved forward with respect to the main body block. In this case, the connection with the main body connecting section can easily be released by moving the replaceable blade forward by moving the slide block forward. Due to this, the replacement of the replaceable blade can more easily be performed.

Further, the slide block is preferably diverged into a center part and a pair of diverged parts arranged at a position on a foreside of the wing section, the center part including the rear-side supporting section and the slide connecting section, and the pair of diverged parts being arranged at both sides with respect to the center part in a height direction, and the rear-side opposing face is formed at fore ends of the pair of diverged parts. In this case, the rear-side opposing face can more stably be made to contact the rear end of the replaceable blade. That is, since the pair of diverged parts is formed at different portions from the center part, the fore ends of the diverged parts can be processed as end surfaces. Due to this, by forming the rear-side opposing face at the fore ends of these diverged parts, the rear-side opposing face can be a face that is parallel to the thickness direction of the replaceable blade. As a result, the rear-side opposing face can stably be made to contact the rear end of the replaceable blade, so that the replaceable blade can stably be pushed out forward when the slide block is moved forward with respect to the main body block.

Preferably, the wing section is formed protruding in both sides in the height direction of the slide block. The front side retaining section is formed at each of both sides in the height direction of the container concave section. Each of the front side retaining sections at the both sides includes the protruding engaging section. In this case, the slide block can be retained stably on the main body block.

Preferably, the cutter includes a cylindrical slide cover that is attached slidably in the longitudinal direction with respect to the main body block and opens in both sides along the longitudinal direction. The slide cover is configured capable of being locked at a foreside position where the replaceable blade is covered and a rear-side position where the replaceable blade is exposed. In this case, in the attached state, since the slide cover covers the replaceable blade by locking the slide cover at the foreside position, user's safety can be ensured, and damage to the replaceable blade can be prevented. Further, when using the cutter, smooth usage of the cutter can be ensured by locking the slide cover at the rear-side position.

Further, the slide cover includes a connecting claw section formed toward inside in the thickness direction. The connecting claw section is configured capable of being elastically displaced in the height direction with respect to a cover main body of the slide cover. The main body block includes a slide groove section formed in the longitudinal direction. A foreside retaining section and a rear-side retaining section that respectively retain the connecting claw section from fore and rear directions are formed at a foreside and a rear-side of the slide groove section. The connecting claw section is slidably arranged in the slide groove section, and connected with the foreside retaining section or the rear-side retaining section so that the slide cover is locked at the foreside position or the rear-side position.

Preferably, the slide groove section includes a foreside groove section that extends from the foreside retaining section toward a foreside and opened toward the foreside of the main body block, and a rear-side groove section that extends from the foreside retaining section toward the rear side and is coupled to the rear-side retaining section, and a communicating section for connection between the foreside retaining section and the foreside groove section is formed at opposite sides of a communicating section for connection between the foreside retaining section and the rear-side groove section in the height direction. In this case, the slide cover can easily be attached and detached to and from the cutter body, and can surely be fixed thereto at the foreside position. That is, by providing the foreside groove section, the connecting claw section can be passed through the foreside groove section, whereby the slide cover can easily be attached and detached with respect to the cutter body in a longitudinal direction thereof. Further, with the communicating section between the foreside retaining section and the foreside groove section and the communicating section between the foreside retaining section and the rear-side groove section being formed at the opposite sides in the height direction from one another, the connecting claw section can easily and surely be arranged more easily at the foreside retaining section. For example, when trying to arrange the connecting claw section at the foreside retaining section by sliding the slide cover from the rear-side position to the foreside position, the connecting claw section can be prevented from passing through the foreside retaining section by momentum thereof.

Further, the slide cover may be configured to include a pair of spring arms capable of elastically deforming in the height direction at both sides in the height direction, and the pair of spring arms extends from a continuing section from the cover main body toward the foreside and is coupled by a coupling section to one another at fore end sections, and the connecting claw section may be formed in the coupling section. In this case, the connecting claw section can easily be displaced in the height direction by the elastic deformation of the spring arms.

Preferably, the slide cover is configured by including a front side pressing section capable of elastically deforming in the thickness direction at a position opposite the flexible section of the slide block when the slide cover is arranged at the rear-side position. In this case, when the slide cover is arranged at the rear-side position, the flexible section of the slide block can be pressed in the thickness direction via the front side pressing section. Due to this, work for the release of the attached state by bending the slide block can easily be performed.

Further, the slide cover may be configured to include a front side opening section that exposes the flexible section at the position that opposes the flexible section of the slide block when the slide cover is arranged at the rear-side position. In this case, when the slide cover is arranged at the rear-side position, the flexible section of the slide block exposed at the front side opening section can be pressed in the thickness direction. Due to this, the work for the release of the attached state by bending the slide block can easily be performed.

### EMBODIMENT

### (First Embodiment)

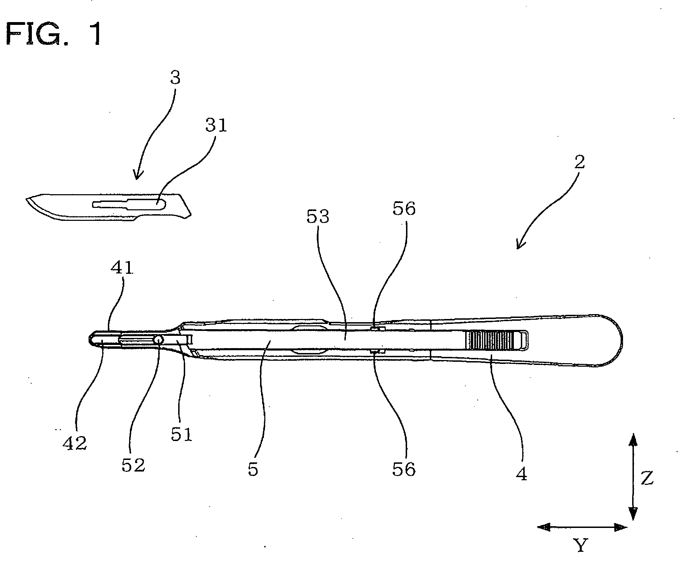

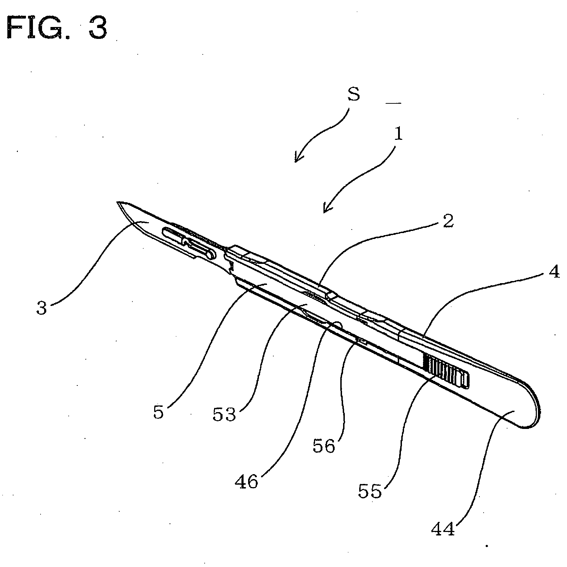

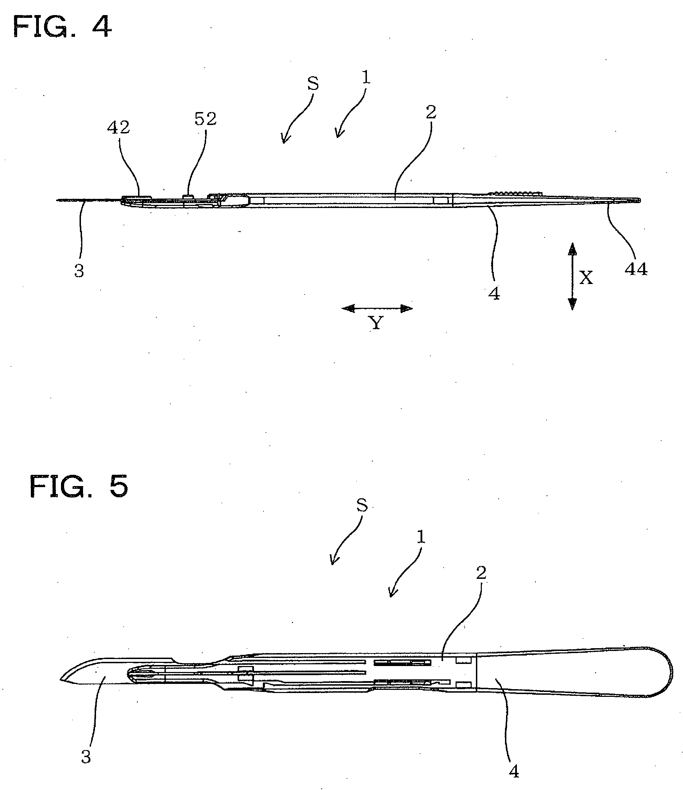

An embodiment of the cutter will be explained with reference to FIG. 1 to FIG. 28.

As shown in FIG. 1 to FIG. 6, a cutter 1 of the present embodiment is a knife for medical use configured by detachably attaching a replaceable blade 3 to a cutter body 2.

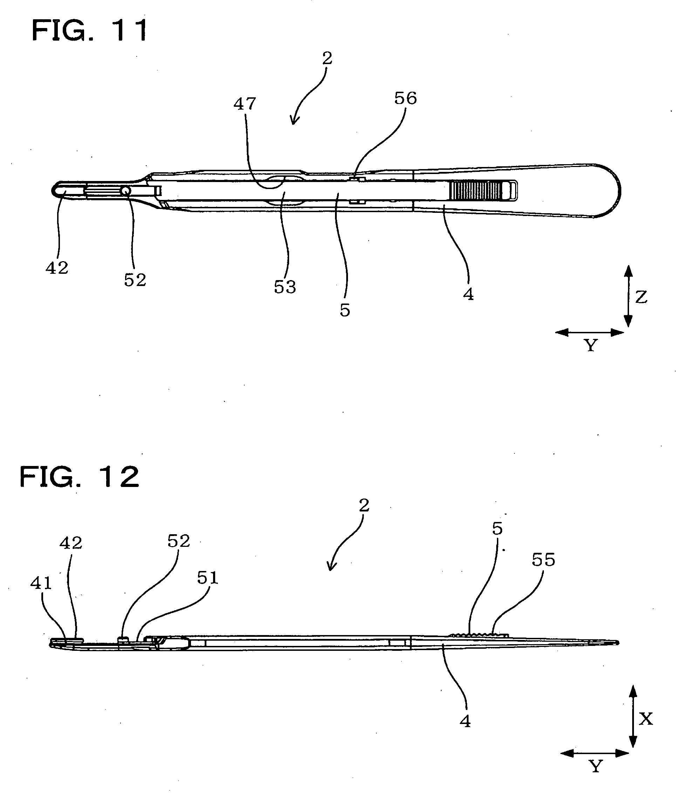

As shown in FIG. 11 to FIG. 13, the cutter body 2 is configured of a main body block 4 and a slide block 5 retained by the main body block 4 so as to be capable of moving in a longitudinal direction Y of the cutter 1.

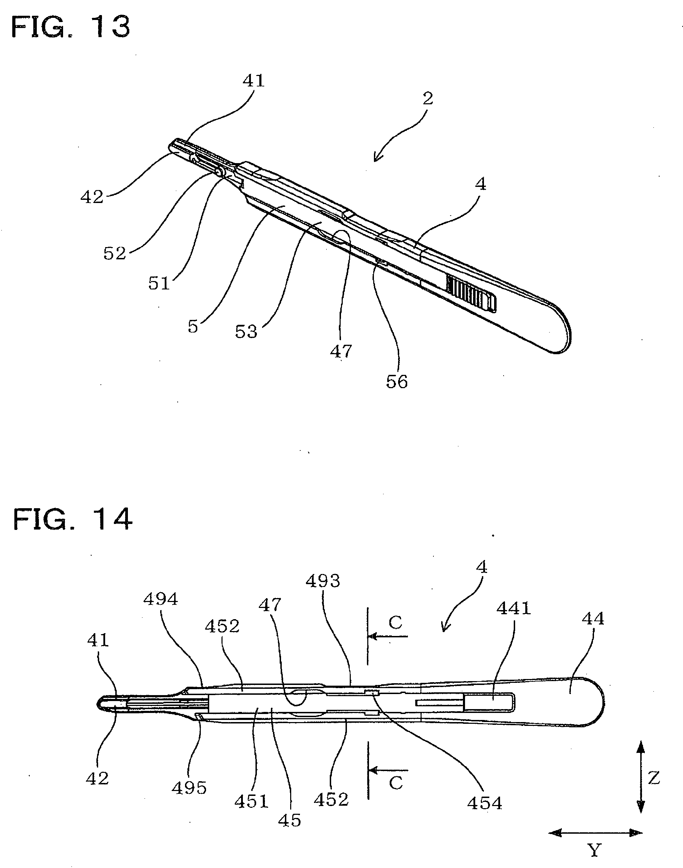

As shown in FIG. 14 to FIG. 18, the main body block 4 has a container section 45 that opens toward a front side and houses the slide block 5 movably in the longitudinal direction Y, a main body supporting section 41 that supports a back face of the replaceable blade 3, and a main body connecting section 42 that connects with the replaceable blade 3 from a front face side.

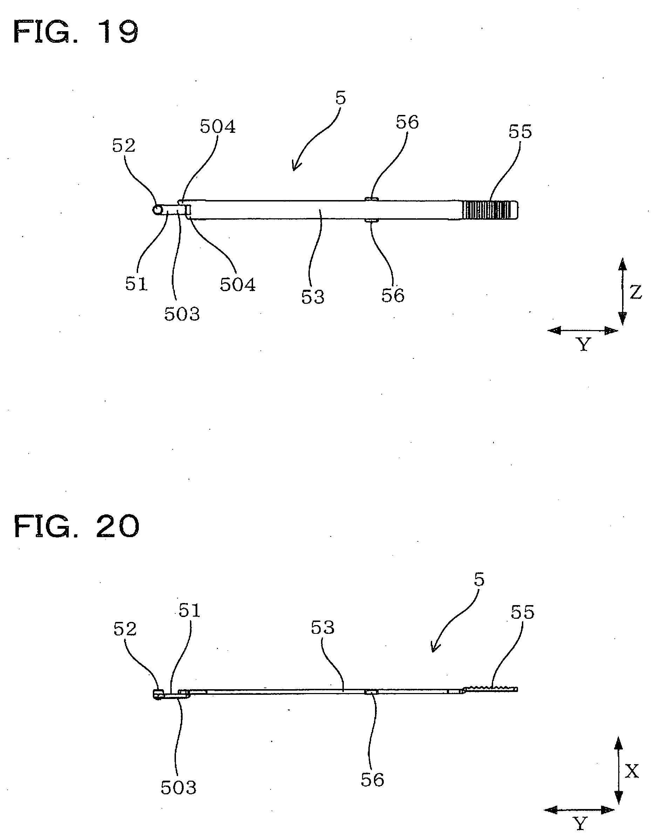

As shown in FIG. 19 to FIG. 21, the slide block 5 has a rear-side supporting section 51 that supports the back face of the replaceable blade 3 at a rear side of the main body supporting section 41, a slide connecting section 52 that connects with the replaceable blade 3 so as to be retracted toward a rear side, a flexible section 53 that bends in a thickness direction X of the replaceable blade 3 at the rear side of the rear-side supporting section 51, and a wing section 56 that protrudes from a part of the flexible section 53 in both sides in a height direction Z.

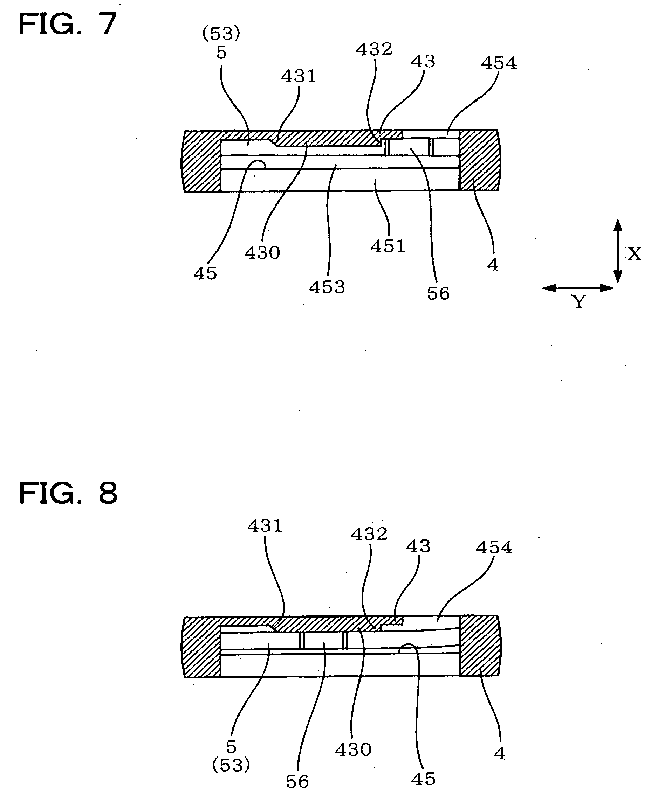

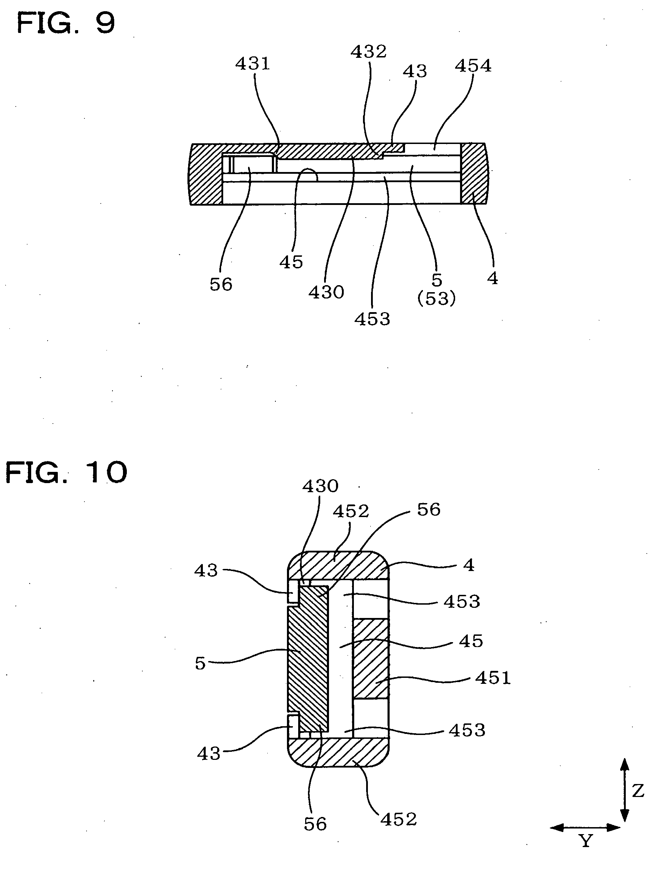

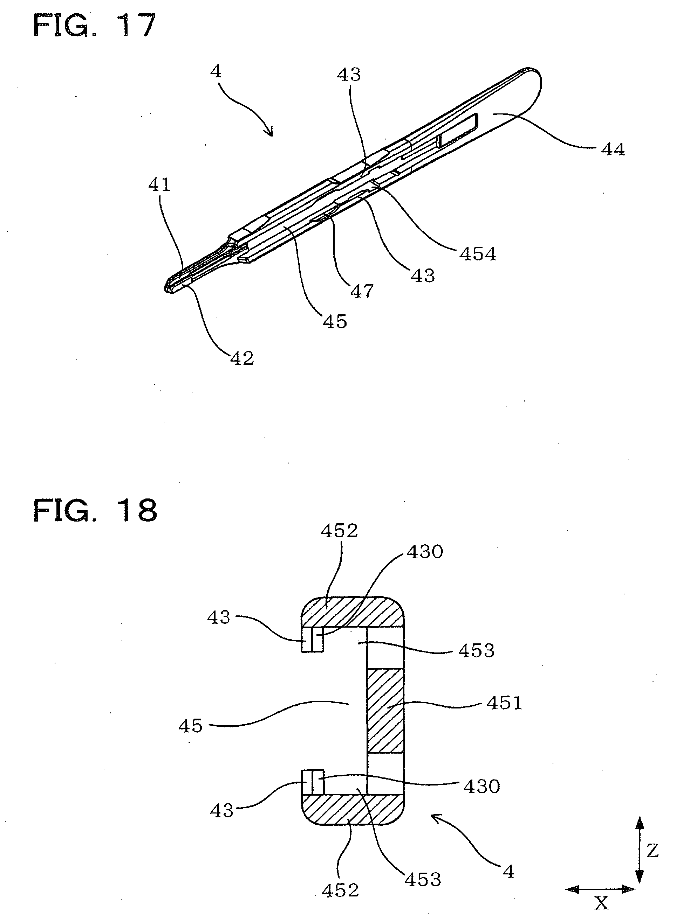

As shown in FIG. 10 and FIG. 18, front side retaining sections 43 that retain the wing section 56 from the front side are formed at both sides in the height direction Z of the container section 45 of the main body block 4. Further, as shown in FIG. 7 to FIG. 10, the front side retaining sections 43 include protruding engaging sections 430 that protrude toward a back face.

As shown in FIG. 7, in an attached state S in which the replaceable blade 3 is attached to the cutter body 2, the wing section 56 is engaged from the foreside by the protruding engaging sections 430. Further, the wing section 56 is configured to be releasable from the protruding engaging sections 430 by bending the flexible section 53 of the slide block 5 to bend in the attached state S.

Note that, in this description, a direction to which a fore end of the replaceable blade 3 points along the longitudinal direction Y of the cutter 1 is expressed as "fore", and an opposite side therefrom as "rear". Further, the thickness direction X refers to a direction that corresponds to a thickness direction of the replaceable blade 3, where a side by which the replaceable blade 3 is supported by the main body supporting section 41 and the rear-side supporting section 51 is referred to as "back", and an opposite side therefrom as "front". Further, a direction that is perpendicular to both the longitudinal direction Y and the thickness direction X is referred to as the height direction Z. The replaceable blade 3 includes a cutting edge 35 at one edge in the height direction Z, where a direction of an edge side onto which the cutting edge 35 is formed in the height direction Z is referred to as "lower side", and an opposite side therefrom as "upper side".

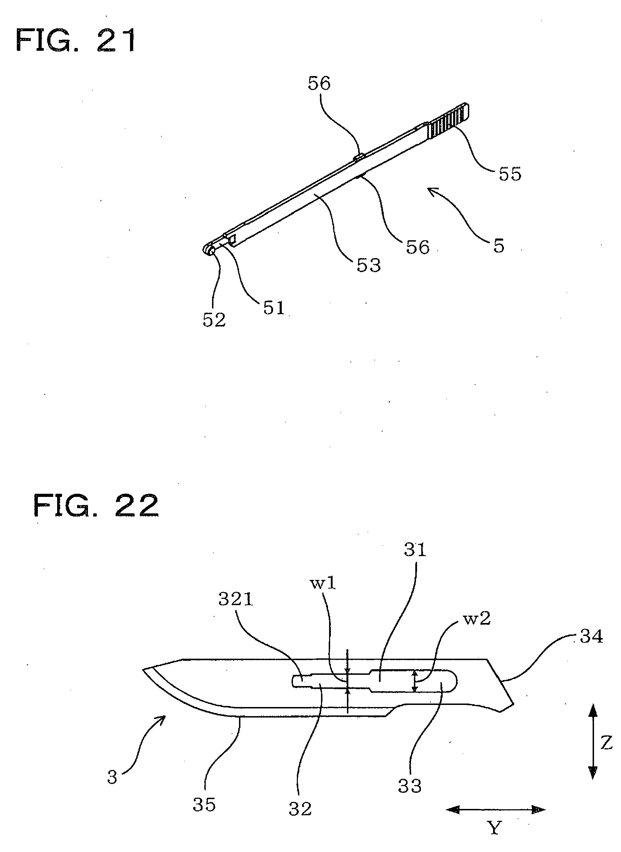

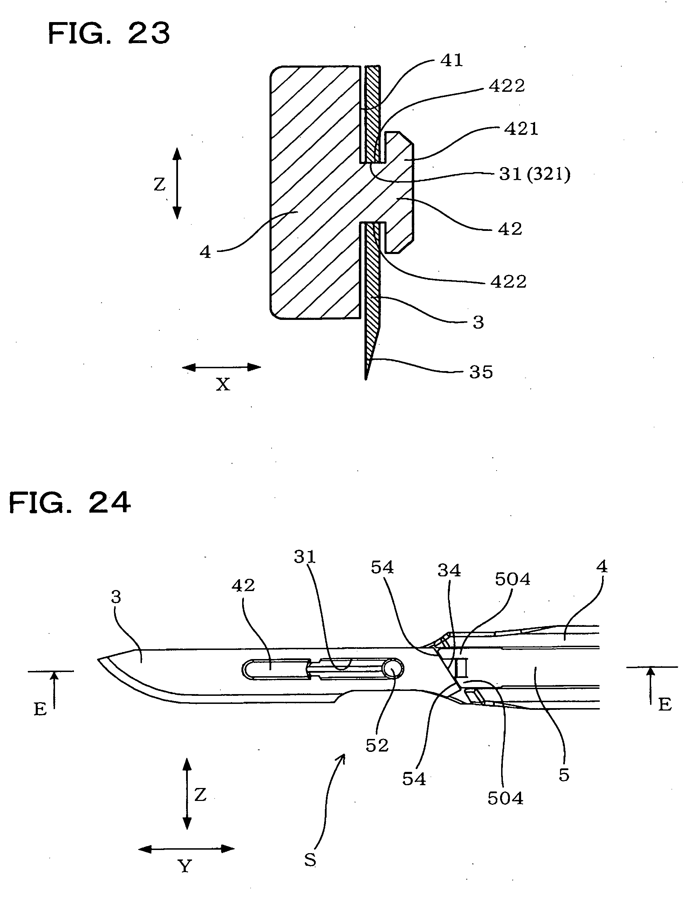

Further, as shown in FIG. 22, the replaceable blade 3 includes an opening section 31 that penetrates in the thickness direction X. As shown in FIG. 23 to FIG. 25, the main body connecting section 42 of the main body block 4 protrudes toward the front side in the thickness direction X from the main body supporting section 41, and is configured to be inserted into the opening section 31 and abut on a fore edge of the opening section 31 in the attached state S. Further, the slide connecting section 52 protrudes toward the front side in the thickness direction X from the rear-side supporting section 51, and is configured to be inserted into the opening section 31 and abut on a rear edge of the opening section 31 in the attached state S.

As shown in FIG. 22, the opening section 31 of the replaceable blade 3 includes a fore end side opening section 32 that is formed at a fore end side and has a relatively small up and down width w1 in the height direction Z perpendicular to the longitudinal direction Y and the thickness direction X of the cutter 1, and a rear end side opening section 33 that is formed continuously on a rear end side of the fore end side opening section 32 and has a larger up and down width w2 than the fore end side opening section 32.

As shown in FIG. 23, the main body connecting section 42 includes a head section 421 having up and down width in the height direction Z smaller than the rear end side opening section 33 and larger than the fore end side opening section 32, and, cut groove sections 422 that are cut from both sides in the height direction Z are formed between the head section 421 and the main body supporting section 41. The replaceable blade 3 is connected with the cut groove sections 422 at an outer periphery of the fore end side opening section 32.

Further, as shown in FIG. 22, at the fore end side of the fore end side opening section 32, a small width portion 321 having a smaller up and down width in the height direction Z is formed. A stable connecting state can be obtained by fitting a fore end section of the main body connecting section 42 of the main body block 4 into the small width portion 321.

Further, the main body supporting section 41 has a larger up and down width in the height direction Z than the rear end side opening section 33.

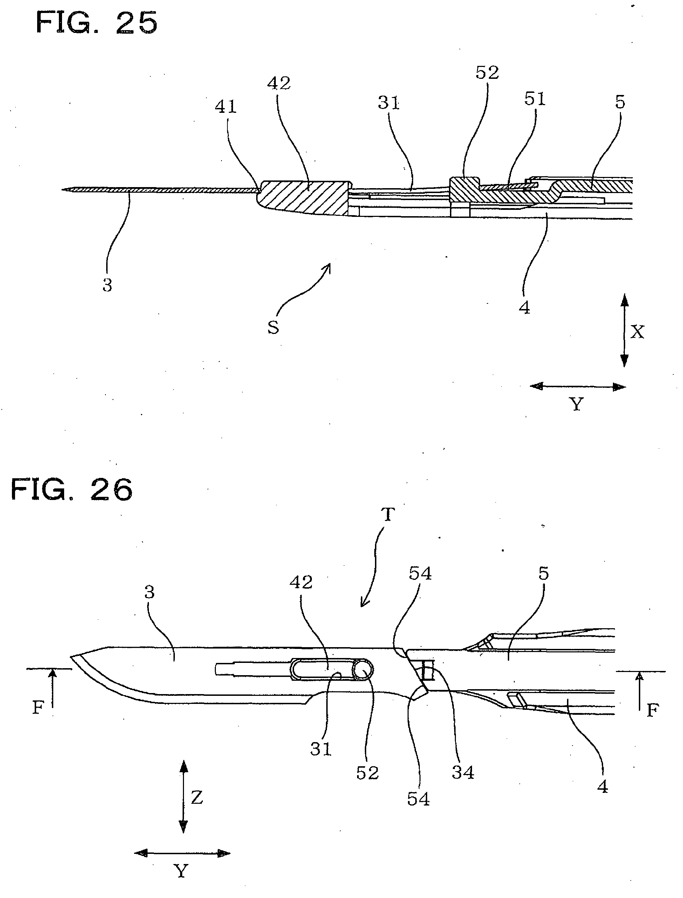

The replaceable blade 3 has flexibility in the thickness direction X. Further, as shown in FIG. 25, in the attached state S, the replaceable blade 3 is elastically deformed so as to curve in the thickness direction X to form a shape protruding toward the backside.

While being in the attached state S, the rear-side supporting section 51 is protruding toward a protruding direction of the backside connecting section 52 farther than the main body supporting section 41.

As shown in FIG. 24, the slide block 5 includes rear side opposing faces 54 that oppose a rear end 34 of the replaceable blade 3 in the longitudinal direction Y. Further, as shown in FIG. 26, when the slide block 5 is moved forward with respect to the main body block 4, the rear-side opposing faces 54 abut on the rear end 34 of the replaceable blade 3 to push the replaceable blade 3 forward.

As shown in FIG. 24, the rear end 34 of the replaceable blade 3 and the rear-side opposing faces 54 are formed tiltingly with respect to the longitudinal direction Y when seen from the thickness direction X. Specifically, the rear end 34 of the replaceable blade 3 is tilted so as to face obliquely upward.

As shown in FIG. 14 to FIG. 17, the main body block 4 of the cutter body 2 includes the main body supporting section 41 and the main body connecting section 42 at the fore end portion, and a grip section 44 at the rear end portion in the longitudinal direction Y. The main body block 4 has the container section 45 that houses the slide block 5 slidably at an inside thereof. As shown in FIG. 18, the container section 45 is defined by a concaved space formed inside a container bottom section 451 formed on the backside in the thickness direction X and container wall sections 452 formed respectively at upper and lower sides in the height direction Z. Further, the front side retaining sections 43 are formed at parts of the pair of container wall sections 452. The front side retaining sections 43 are formed on the front sides of wing section arranging sections 453 that are formed by cutting parts of the container wall sections 452 from a container section 45 side. That is, the front side retaining sections 43 are formed so as to cover the wing section arranging sections 453 from the front side.

At a rear end side of the front side retaining sections 43 in the container wall sections 452, wing section inserting sections 454 that are cut in the height direction Z are formed adjacently. The wing section inserting sections 454 are formed for each pair of container wall sections 452. The slide block 5 is configured to be inserted and arranged in the container section 45 of the main body block 4 from the front side and capable of being detached from the container section 45 under a state in which the pair of wing sections 56 is aligned with respect to the pair of wing section inserting sections 454.

As shown in FIG. 7 to FIG. 9, the front side retaining sections 43 include the protruding engaging sections 430 protruding toward the wing section arranging sections 453 at their backside face. Rear end sections 432 of the protruding engaging sections 430 are arranged at the foreside of fore ends of the wing section inserting sections 454, and fore end sections 431 of the protruding engaging sections 430 are arranged at the rear-side of fore ends of the wing section arranging sections 453.

The fore end sections 431 of the protruding engaging sections 430 include tapered sections whose protruding amount decreases toward the fore end side. On the other hand, the rear end sections 432 of the protruding engaging sections 430 include end faces that are perpendicular to the longitudinal direction Y. As shown in FIG. 7, in the attached state S, the wing sections 56 of the slide block 5 are engaged while abutting on the end faces of the rear end sections 432 of the protruding engaging sections 430. Further, as shown in FIG. 9, in a blade replacing state T, the wing sections 56 of the slide block 5 are arranged at a foreside of the fore end sections 431 of the protruding engaging sections 430. More accurately, the wing sections 56 have their corner sections at the rear side thereof abutted on the tapered sections at the fore end sections 431 of the protruding engaging sections 430. Further, when moving the slide block 5 between the attached state S and the blade replacing state T, the flexible section 53 of the slide block 5 is made to bend, whereby the pair of wing sections 56 are allowed to pass over the protruding engaging sections 430 from their back face side.

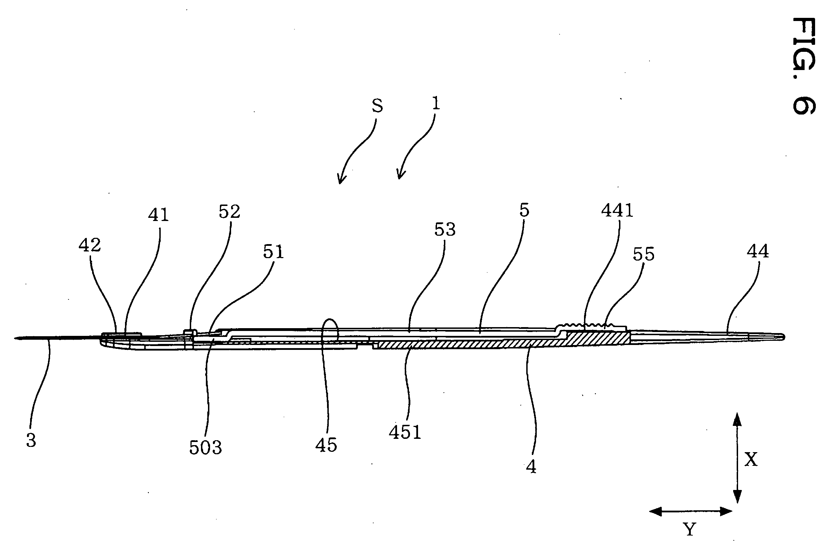

As shown in FIG. 19 to FIG. 21, the slide block 5 includes the rear-side supporting section 51 and the slide connecting section 52 at the fore end portion, and an operating section 55 at the rear end portion in the longitudinal direction Y. The operating section 55 rises toward the front side and extends rearward. A concavo-convex section for preventing slippage with a finger of a user (operator) is formed at a front face of the operating section 55. As shown in FIG. 6, the operating section 55 is slidably mounted on a concaved mounting face 441, that is a part of the grip section 44 and provided at a portion adjacent to the rear end of the container section 45 in the main body block 4.

As shown in FIG. 19, the slide block 5 is diverged into a center part 503 and a pair of diverged parts 504 at a position to the foreside of the wing sections 56, where the pair of diverged parts 504 is arranged at both sides with respect to the center part 503 in the height direction Z.

The center part 503 includes the rear-side supporting section 51 and the slide connecting section 52. That is, the rear-side supporting section 51 is formed so as to extend forward at a position retracted toward the back face side in the thickness direction X from a diverging portion, and the slide connecting section 52 is formed at the fore end section of the rear-side supporting section 51 so as to protrude toward the front face side in the thickness direction X.

The pair of diverged parts 504 is arranged at both sides with respect to the center part 503 in the height direction Z, and extends forward. Further, as shown in FIG. 24, the rear-side opposing faces 54 that oppose the rear end 34 of the replaceable blade 3 are formed at fore ends of this pair of diverged parts 504.

Of the pair of diverged parts 504, the diverged part 504 on the upper side of the height direction Z is longer than the diverged part 504 on the lower side. Further, the rear-side opposing faces 54 formed respectively thereon are formed in substantially the same plane. Further, the rear-side opposing faces 54 are tilted with respect to the longitudinal direction Y and the height direction Z, and are formed parallel to the thickness direction X. That is, the pair of rear-side opposing faces 54 is formed so as to be substantially parallel to the tilted rear end 34 of the replaceable blade 3.

Further, the rear-side opposing faces 54 of the diverged parts 504 are formed as end surfaces of the slide block 5. That is, when punching or machining the slide block 5, the rear-side opposing faces 54 are formed as processed end surfaces therein.

As shown in FIG. 26, when the slide block 5 is moved forward with respect to the main body block 4, the replaceable blade 3 is configured to be pushed forward by the pair of rear-side opposing faces 54, pressing the rear end 34 of the replaceable blade 3.

Further, the slide block 5 needs to be configured to allow the flexible section 53 to be bent in the thickness direction X while protruding toward the backside in the state where the slide block 5 is housed in the container section 45 of the main body block 4. Due to this, as shown in FIG. 6, FIG. 7, and FIG. 10, a gap is formed between the back face of the flexible section 53 of the slide block 5 and the container bottom section 451 of the container section 45 of the main body block 4 when the slide block 5 is housed in the container section 45. Further, the slide block 5 abuts on the main body block 4 at the back face in the vicinity of the fore end section and the rear end section. Specifically, a back face of the center part 503 in the vicinity of the fore end section and a back face of the operating section 55 at the back end section of the slide block 5 abut on the main body block 4, and the gap is formed between the back face of the portion defined by the center part 503 and the operating section 55, and the main body block 4.

In other words, in the present embodiment, a portion between the center part 503 and the operating section 55 can be said to be serving as the flexible section 53.

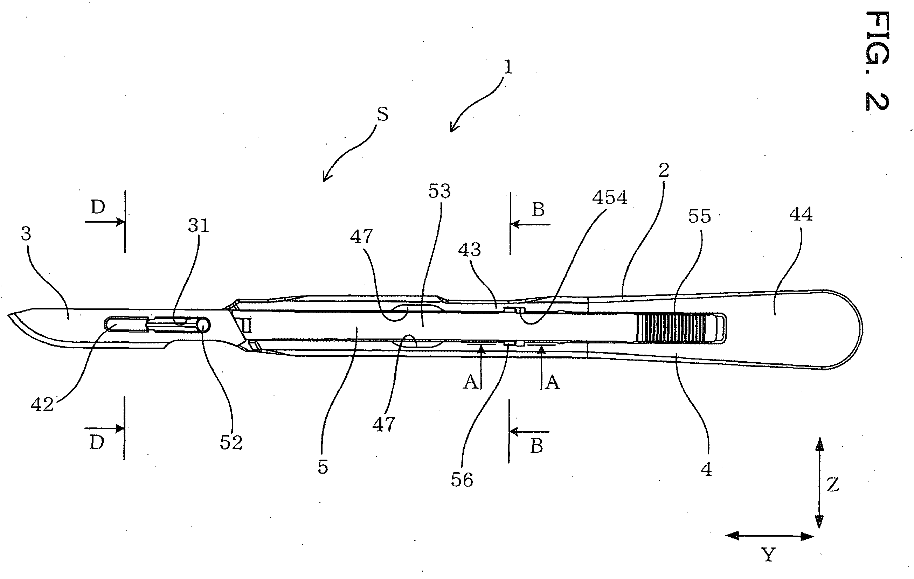

Further, as shown in FIG. 2, FIG. 14 and FIG. 17, front face concaved sections 47 that are adjacent at upper and lower sides in the height direction Z with respect to the container section 45 are formed on the front side face of the main body block 4. The front face concaved sections 47 are formed adjacent to a part of the flexible section 53 of the slide block 5 housed in the container concaved section 45. Due to this, the opening section of the container section 45 between the pair of front face concaved sections 47 is widened, and this portion makes it easier for the user to press the flexible section 53 of the slide block 5 with fingers.

Next, a method of attaching and a method of detaching the replaceable blade 3 in the cutter 1 of this embodiment will be explained.

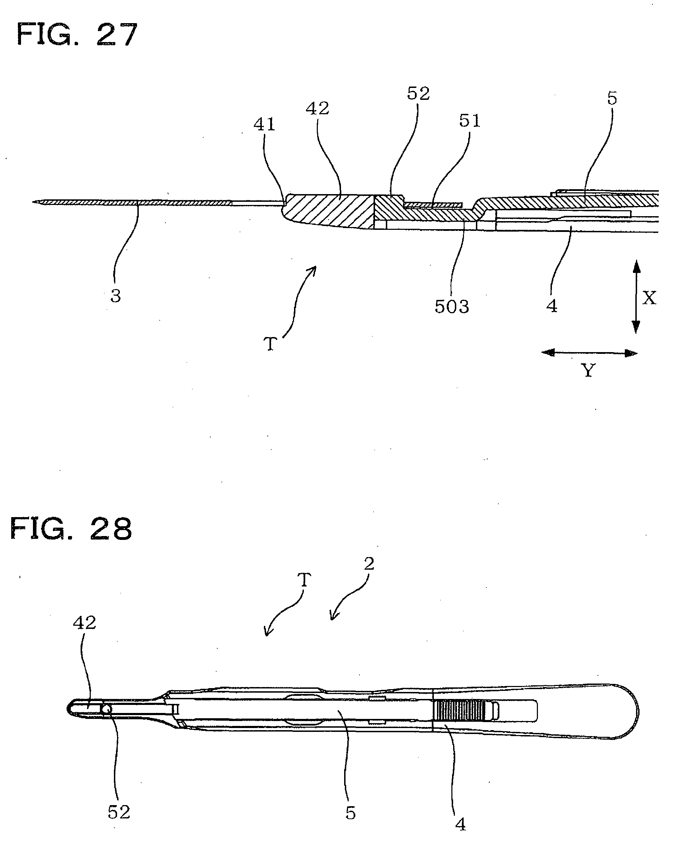

Firstly, as shown in FIG. 28, the slide block 5 is moved forward, and the cutter body 2 is made to be in the blade replacing state T. In this state, the main body connecting section 42 of the main body block 4 and the slide connecting section 52 of the slide block 5 are connected. The replaceable blade 3 is arranged at the fore end section of the cutter body 2 as shown in FIG. 26 with the connected main body connecting section 42 and slide connecting section 52 passing through the rear end side opening section 33 of the replaceable blade 3.

At this occasion, the back face of the replaceable blade 3 is brought into contact with the main body supporting section 41 of the main body block 4 and the rear-side supporting section 51 of the slide block 5.

Further, the rear end 34 of the replaceable blade 3 is arranged opposite the rear-side opposing faces 54 of the slide block 5.

Next, the slide block 5 is moved rearward with respect to the main body block 4. At this occasion, as shown in FIG. 7 to FIG. 9, the pair of wing sections 56 of the slide block 5 slidably moves from the fore end sections 431 of the pair of protruding engaging sections 430 of the main body block 4 on the back faces thereof toward the rear-side. That is, the wing sections 56 move to the back faces of the protruding engaging sections 430 along the tapered sections of the fore end sections 431 of the protruding engaging sections 430. In accompanying this, the slide block 5 bends in the protruding shape toward the back face side at the flexible section 53. Although the bending can be achieved by pressing the flexible section 53 in toward the back face side when retracting the slide block 5 rearward, as described above, there is no need for a beforehand bending since the slide block 5 naturally bends in the course of moving the slide block 5 rearward in the presence of the tapered sections formed in the fore end sections 431 of the protruding engaging sections 430.

As shown in FIG. 24, by retracting the slide block 5 rearward as described above, the slide connecting section 52 pulls the replaceable blade 3 rearward at the rear end of the rear end side opening section 33. Then, as the replaceable blade 3 moves toward the rear-side, the main body connecting section 42 moves relatively toward the fore side opening section 32 of the replaceable blade 3. Due to this, as shown in FIG. 23, the replaceable blade 3 is connected to the cut groove sections 422 provided in the main body connecting section 42 at upper and lower portions of the fore end side opening section 32. Then, by further pulling the slide block 5 farther in the rear-side with respect to the main body block 4, as shown in FIG. 24 and FIG. 25, the fore end of the main body connecting section 42 is fitted into the small width portion 321 of the fore end side opening section 32 of the replaceable blade 3, and makes contact with a fore end thereof.

Further, as shown in FIG. 7 to FIG. 9, the wing sections 56 of the slide block 5 slides rearward on the back faces of the protruding engaging sections 430, and reach the rear end sections 432 of the protruding engaging sections 430. At least the flexible section 53 of the slide block 5 is elastically deformed so as to bend protrudingly toward the backside while the wing sections 56 are sliding on the back faces of the protruding engaging section 430, and the wing sections 56 are biased toward the front side. Due to this, when the wing sections 56 of the slide block 5 reach the rear end sections 432 of the protruding engaging sections 430, the wing sections 56 move naturally toward the front side by their restoring force, and are engaged with the rear end sections 432 of the protruding engaging sections 430. That is, the wing sections 56 are restricted to move forward in the longitudinal direction Y.

Further, the wing sections 56 are at least partially retained by the front side retaining sections 43 at the rear side of the protruding engaging sections 430 from the front side.

Accordingly, the attached state S in which the replaceable blade 3 is attached to the cutter body 2 is realized.

As shown in FIG. 25, in this attached state S, the replaceable blade 3 is elastically deformed in the state of being bent protrudingly toward the backside. That is, in the attached state S, the rear-side supporting section 51 of the slide block 5 protrudes toward the front side farther than the main body supporting section 41 of the main body block 4. At this occasion, the slide block 5 is not in a state of particularly being bent.

Further, when detaching the replaceable blade 3 from the cutter body 2, the slide block 5 is moved forward with respect to the main body block 4 from the attached state S. At this occasion, the slide block 5 is pushed in toward the backside at the flexible section 53 to be bent thereat. Due to this, the pair of wing sections 56 is displaced toward the backside farther than the protruding engaging sections 430 of the main body block 4, and the connected state of the wing section 56 and the protruding engaging sections 430 is released. As shown in FIG. 7 to FIG. 9, by pressing the slide block 5 in toward the foreside with respect to the main body block 4 in this state, the wing sections 56 move toward the foreside by sliding on the back faces of the protruding engaging sections 430.

Then, as the slide block 5 is moved forward, as shown in FIG. 26, the replaceable blade 3 is moved forward by the rear-side opposing faces 54 provided in the slide block 5 while pressing the rear end 34 of the replaceable blade 3 forward. Due to this, the main body connecting section 42 of the main body block 4 relatively moves rearward in the opening section 31 of the replaceable blade 3, and shifts from the fore end side opening section 32 to the rear end side opening section 33. Then, when the slide block 5 is moved forward until the slide connecting section 52 abuts on the main body connecting section 42, both the main body connecting section 42 and the slide connecting section 52 are arranged at the rear end side opening section 33. In this state, the replaceable blade 3 is detached toward the front side.

The replaceable blade 3, the main body block 4 and the slide block 5 are all made of metal such as stainless steel.

Next, an advantageous effect of the present embodiment will be explained.

In the aforementioned cutter 1, when attaching the replaceable blade 3 to the cutter body 2, the slide block 5 is moved forward so as to realize the blade replacing state T in which the replaceable blade 3 can be attached and detached, and the replaceable blade 3 is temporarily engaged with the main body connecting section 42 and the slide connecting section 52. Then, the main body connecting section 42 and the slide connecting section 52 are caused to pull the replaceable blade 3 in both sides in the longitudinal direction by moving the slide block 5 rearward. Due to this, the attached state S in which the replaceable blade 3 is attached to the cutter body 2 can be realized.

Further, contrary to the above, the replacement of only the replaceable blade 3 is easily enabled by realizing the blade replacing state T from the attached state S by forcibly moving the slide block 5 forward.

Further, as shown in FIG. 7, in the attached state S, the wing sections 56 are engaged from the foreside by the protruding engaging sections 430. Due to this, the slide block 5 is restricted from moving forward, and the attached state S is maintained. Further, the wing sections 56 are configured capable of being released from the protruding engaging sections 430 by causing the flexible section 53 of the slide block 5 to bend from the attached state S.

That is, the cutter 1 can easily realize fixation of the slide block 5 in the attached state S and switching from the attached state S to the blade replacing state T by utilizing the elastic deformation of the flexible section 53 of the slide block 5. Due to this, no additional member for restricting the slide block 5 to move forward in the attached state S is required, and a configuration for releasing the attached state S can be significantly simplified.

Due to this, the cutter 1 can decrease a number of components, and have its configuration simplified.

Further, the protruding engaging sections 430 includes the tapered section at the fore end sections 431. Due to this, the wing sections 56 may be moved on the back faces of the protruding engaging sections 430 along the tapered sections upon sliding the slide block 5 from the blade replacing state T to the attached state S. Subsequent to the movement, the slide block 5 is further moved rearward so as to easily engage the wing sections 56 with the rear end sections 432 of the protruding engaging sections 430. Thus, the slide block 5 is allowed to move smoothly from the blade replacing state T to the attached state S.

Further, the replaceable blade 3 has flexibility in the thickness direction X, and elastically deforms in the attached state S so as to curve protrudingly toward the backside in the thickness direction. Due to this, the replaceable blade 3 is pressed against the main body supporting section 41, the main body connecting section 42, and the rear-side supporting section 51 by the elastic force (restoring force) of itself. Due to this, the replaceable blade 3 can more stably be fixed to the cutter body 2.

Further, in the attached state S, the rear-side supporting section 51 protrudes toward the front side in the thickness direction X farther than the main body supporting section 41. Due to this, the replaceable blade 3 that is engaged with the main body connecting section 42 and is supported at its back face by the main body supporting section 41 and the rear-side supporting section 51 can easily curve in the protruding shape toward the main body supporting section 41 and the rear-side supporting section 51. Due to this, the replaceable blade 3 can surely be fixed to the cutter body 2 by the elastic force of the replaceable blade 3.

Further, in the attached state S, the main body connecting section 42 is inserted in the opening section 31 of the replaceable blade 3 and abuts on the fore edge of the opening section 31, and the slide connecting section 52 is inserted in the opening section 31 of the replaceable blade 3 and abuts on the rear edge of the opening section 31. Due to this, the replaceable blade 3 can easily and surely be connected by the main body connecting section 42 and the slide connecting section 52, and the attached state S can easily be formed.

Further, the slide block 5 includes the rear-side opposing faces 54, and the rear-side opposing faces 54 are configured to press the replaceable blade 3 toward the foreside by the rear-side opposing faces 54 while abutting on the rear end 34 of the replaceable blade 3 when the slide block 5 is moved forward with respect to the main body block 4. Due to this, the connection with the main body connecting section 42 can easily be released by moving the slide block 5 forward to move the replaceable blade 3 forward. Due to this, the replacement of the replaceable blade can more easily be performed.

Further, the slide block 5 is diverged into the center part 503 and the pair of diverged parts 504, the rear-side opposing faces 54 are formed at the fore ends of the pair of diverged parts 504. Due to this, the rear-side opposing faces 54 can more stably be abutted on the rear end 34 of the replaceable blade 3. That is, since the pair of diverged parts 504 is formed at different portions from the center part 503, the fore ends of the diverged parts 504 can be processed as end surfaces. Due to this, by forming the rear end side opposing faces 54 at the fore ends of these diverged parts 504, the rear-side opposing faces 54 can be those parallel to the thickness direction X of the replaceable blade 3. As a result, the rear-side opposing faces 54 can stably be abutted on the rear end 34 of the replaceable blade 3, so that the replaceable blade 3 can stably be pushed forward when the slide block 5 is moved forward with respect to the main body block 4.

As described above, according to the present embodiment, the replacement of only the replaceable blade is enabled while providing the blade with simple configuration with less number of components.

### (Second Embodiment)

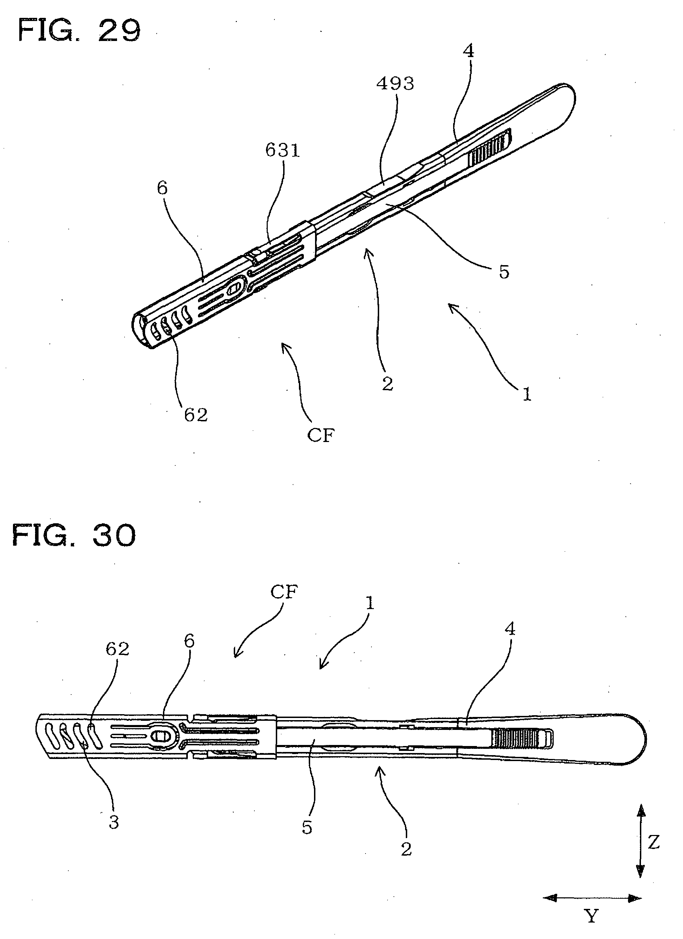

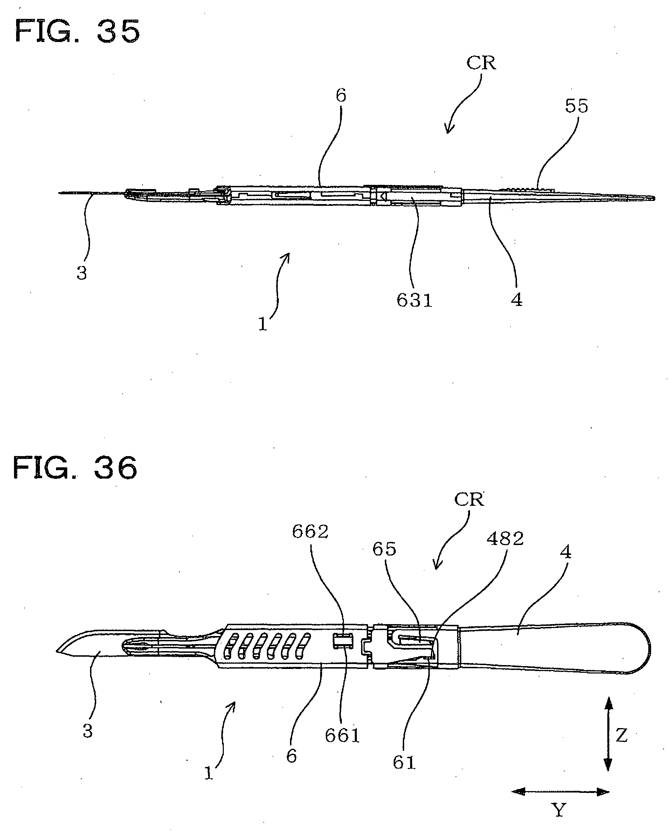

As shown in FIG. 29 to FIG. 41, the present embodiment is formed as the cutter 1 having a slide cover 6 slidably attached in a longitudinal direction Y with respect to the main body block 4. Since configurations other than the slide cover 6 are similar to those in the first embodiment, the explanation of this embodiment will be made by using FIG. 1 to FIG. 28 as appropriate.

The slide cover 6 has a cylindrical shape that opens in both sides along the longitudinal direction. The slide cover 6 is made of metal such as stainless steel, etc., and is formed by cutting and bending a single metal plate.

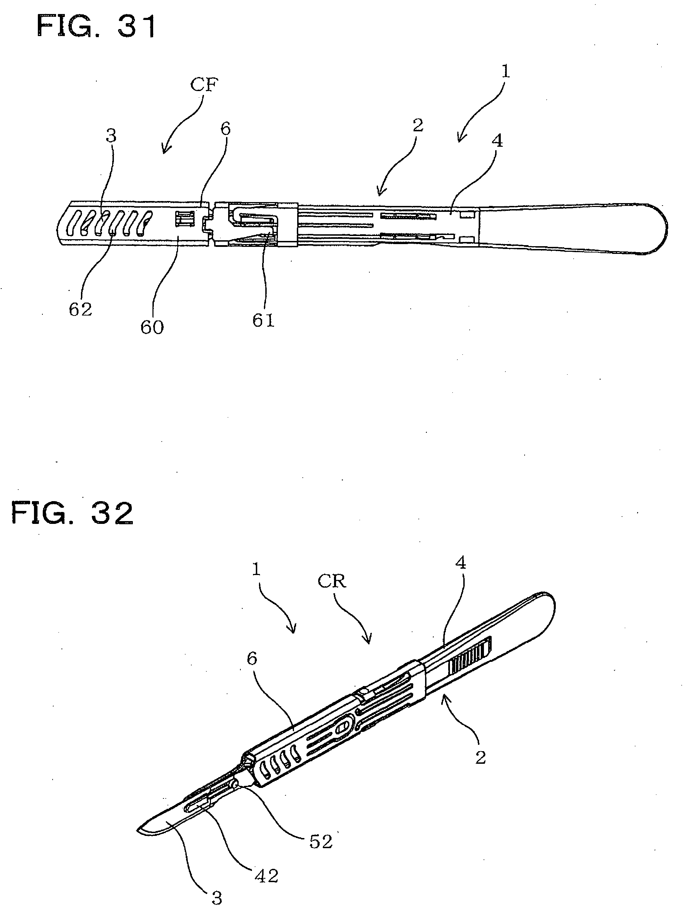

The slide cover 6 is configured to be locked at a foreside position CF covering the replaceable blade 3 (FIG. 29 to FIG. 31) and a rear-side position CR exposing the replaceable blade 3 (FIG. 32 to FIG. 36).

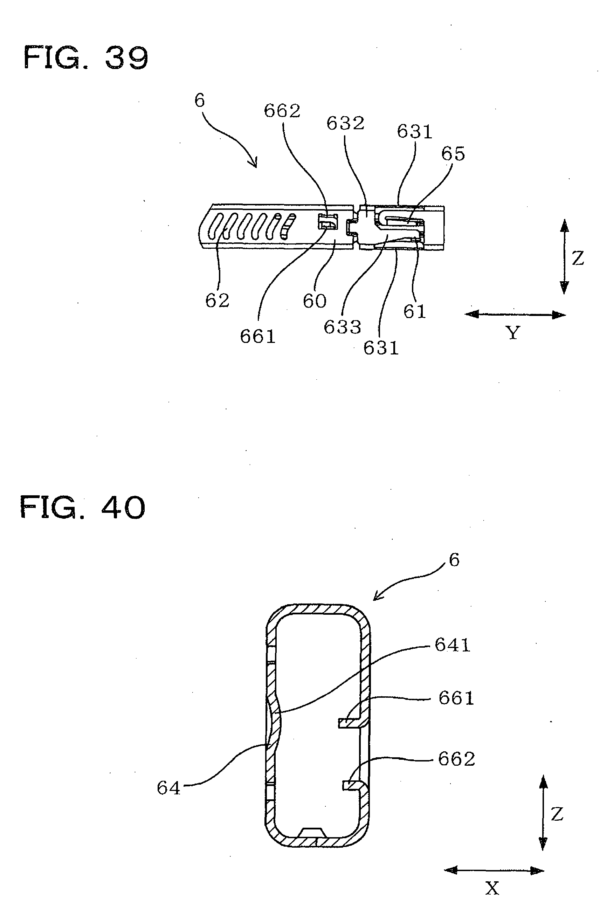

As shown in FIG. 39, the slide cover 6 includes a connecting claw section 61 formed toward inside in a thickness direction X, and the connecting claw section 61 is configured to displace elastically in the height direction Z with respect to a cover main body 60 of the slide cover 6. The connecting claw section 61 protrudes toward a front side (inside) from a backside of the slide cover 6.

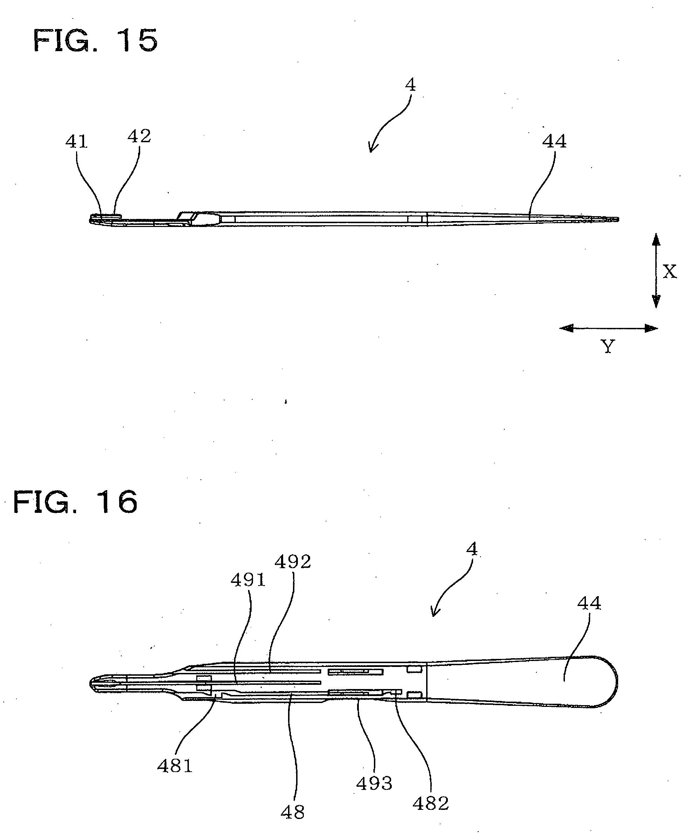

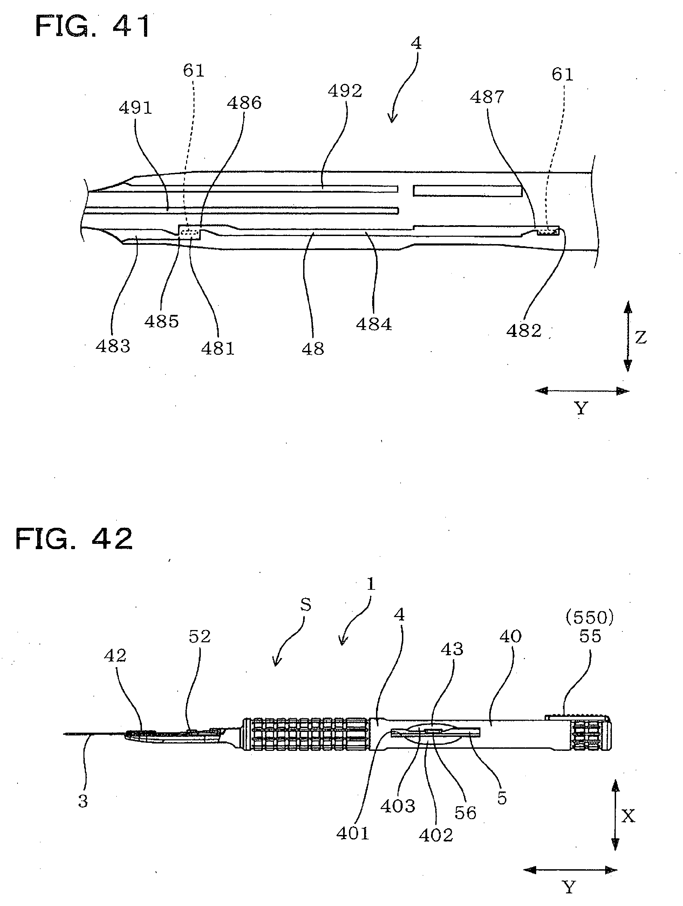

As shown in FIG. 16 and FIG. 41, the main body block 4 includes a slide groove section 48 formed in the longitudinal direction Y at a back face thereof. A foreside retaining section 481 and a rear-side retaining section 482 that respectively retain the connecting claw section 61 from fore and rear directions are formed at a foreside and a rear-side of the slide groove section 48.

The connecting claw section 61 is arranged slidably in the slide groove section 48. Further, the slide cover 6 is locked at the foreside position CF or the rear-side position CR by connecting the connecting claw section 61 with the foreside retaining section 481 or the rear-side retaining section 482, respectively.

The slide groove section 48 includes a foreside groove section 483 that extends from the foreside retaining section 481 toward the foreside and opened toward the foreside of the main body block 4, and a rear-side groove section 484 that extends from the foreside retaining section 481 toward the rear side and coupled to the rear-side retaining section 482.

That is, the slide groove section 48 is configured of the foreside groove section 483, the foreside retaining section 481, the rear-side groove section 484, and the rear-side retaining section 482 arranged in this order from the foreside. They are continuously arranged to form a substantially straight line in the longitudinal direction Y. Note that, communicating sections 485, 486 and 487 that are constricted from any sides in the height direction Z communicate the foreside groove section 483 and the foreside retaining section 481 as well as the foreside retaining section 481 and the rear-side groove section 484 and also the rear-side groove section 484 and the rear-side retaining section 482. The communicating sections 486 and 487 are constricted from the upper side (which is a lower side in FIG. 41) in the height direction Z. On the other hand, the communicating section 485 is constricted from the lower side (which is an upper side in FIG. 41) in the height direction Z.

That is, the communicating section 485 between the foreside retaining section 481 and the foreside groove section 483 and the communicating section 486 between the foreside retaining section 481 and the rear-side groove section 484 are formed in opposite sides from one another in the height direction Z.

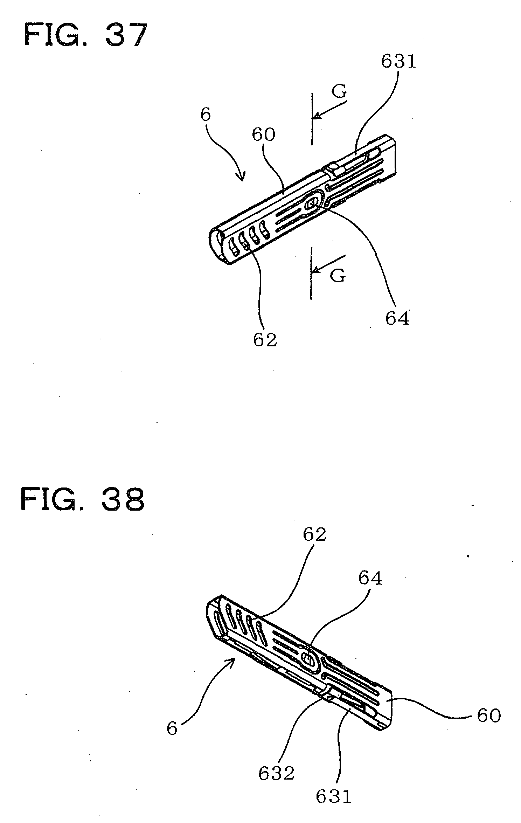

As shown in FIG. 37 to FIG. 39, the slide cover 6 includes a pair of spring arms 631 capable of elastically deforming in the height direction Z at both sides in the height direction Z. The pair of spring arms 631 extends from a continuing section from the cover main body 60 toward the foreside and is coupled by a coupling section 632 to one another at fore end sections. The connecting claw section 61 is formed in this coupling section 632. Specifically, the connecting claw section 61 is formed by bending a fore end portion of an elongating section 633 extending rearward from the coupling section 632 at about 90 degrees toward the front side with respect to the axis of the longitudinal direction Y.

Further, as shown in FIG. 14, a constricted section 493 that is concaved toward the lower side is formed at a portion as a part of an upper face of the main body block 4 where the upper spring arm 631 is to be arranged opposingly when the slide cover 6 is positioned at the rear-side position CR. Due to this, as will be described later, the upper side spring arm 631 is allowed to elastically deform downward at the rear-side position CR. Further, an upper side tilted section 494 that is concaved toward the lower side at a greater degree as it extends forward is formed at a portion as a part of the upper face of the main body block 4 and where the upper spring arm 631 is to be arranged opposingly when the slide cover 6 is positioned at the foreside position CF. Further, a lower side tilted section 495 that is conclaved toward the upper side at a greater degree as it extends forward is formed at a portion as a part of a lower face of the main body block 4 where the lower spring arm 631 is to be arranged opposingly when the slide cover 6 is positioned at the foreside position CF. As will be described later, the upper and lower spring arms 631 are allowed to elastically deform in the lower or upper side at the foreside position CF in the presence of these upper side tilted section 494 and lower side tilted section 495.

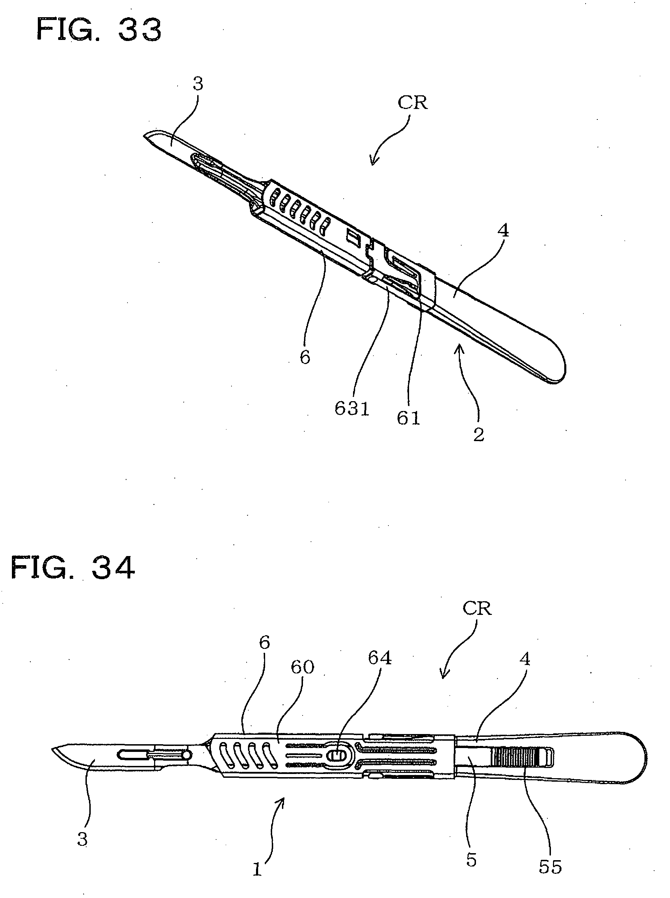

Further, as shown in FIG. 34, FIG. 37 and FIG. 38, the slide cover 6 includes a front side pressing section 64 capable of elastically deforming in the thickness direction X at a position that opposes the flexible section 53 of the slide block 5 when it is arranged at the rear-side position CR.

The front side pressing section 64 is formed on the front side of the slide cover 6, extends from the continuing section with the cover main body 60 toward the rear side, and has its rear end section configured capable of significantly deforming in the thickness direction X. As shown in FIG. 40, the front side pressing section 64 includes a curved section 641 that curves toward the inside (backside) in the thickness direction X, and abuts on the flexible section 53 of the slide block 5 at this curved section 641.

Further, as shown in FIG. 40, aside from the connecting claw section 61, the slide cover 6 includes two sliding claw sections 661 and 662 that stand toward the inside from the backside. The sliding claw sections 661 and 662 are respectively arranged in two sliding groove sections 491 and 492 formed on the back face of the main body block 4 as shown in FIG. 16 and FIG. 41. The sliding groove sections 491 and 492 are formed at the back face of the main body block 4 parallel to the longitudinal direction Y, and are opened toward the foreside. Further, when the slide cover 6 is slid back and forth with respect to the main body block 4, the sliding claw sections 661 and 662 respectively slide back and forth in the sliding groove sections 491 and 492.

Further, as shown in FIG. 39, a presser spring section 65 that is capable of elastically deforming in the thickness direction X is formed at the backside of the slide cover 6. The presser spring section 65 abuts elastically on the back face of the main body block 4, and prevents looseness of the slide cover 6 in the thickness direction X with respect to the main body block 4.

Further, as shown in FIG. 37 to FIG. 39, the slide cover 6 has a plurality of window sections 62 at both the front side and the back side, which penetrate the inside of the slide cover 6. As shown in FIG. 29 to FIG. 31, even when the slide cover 6 is arranged at the foreside position CF and the state in which the replaceable blade 3 is covered is realized, the replaceable blade 3 can be visibly recognized from the window sections 62 through the window sections 62. Further, in sterilizing the cutter 1, since sterilizing solution can pass through the window sections 62, sterilization of the replaceable blade 3 and the fore end portion of the cutter body 2 can effectively be performed.

Meanwhile, when using the cutter 1, as shown in FIG. 32 to FIG. 36, the slide cover 6 is locked at the rear-side position CR. Due to this, the replaceable blade 3 is exposed from the slide cover 6.

At this occasion, the connecting claw section 61 of the slide cover 6 is connected with the rear-side retaining section 482 provided at the rear end of the slide groove section 48 of the main body block 4. Due to this, the slide cover 6 at the rear-side position CR is restricted to move forward with respect to the main body block 4.

Next, when sliding the slide cover 6 from the rear-side position CR to the foreside position CF, the connecting claw section 61 is lowered downward in the height direction Z (upper side in FIG. 41) by pressing the upper side spring arm 631 of the pair of spring arms 631 downward. Due to this, the connecting claw section 61 is detached from the rear-side retaining section 482, and is allowed to move forward from the communicating section 487. Further, the slide cover 6 is slid forward. At this occasion, the connecting claw section 61 reaches the foreside retaining section 481 bypassing through the rear-side groove section 484 of the slide groove section 48. The connecting claw section 61 is arranged from the communicating section 486 to the foreside retaining section 481. In this state, the pair of spring arms 631 is brought into a free state in which no bias in the height direction Z is imposed. Then, at the foreside retaining section 481, the connecting claw section 61 is positioned at a substantial center portion in the height direction Z. This position is at the side lower than the communicating section 485 at the foreside of the foreside retaining section 481, and is at the side upper than the communicating section 486 at the rear side of the foreside retaining section 481. That is, at the foreside retaining section 481, the connecting claw section 61 is brought into a state where its movement in the back and forth direction is restricted.

Accordingly, the slide cover 6 is locked at the foreside position CF.

Then, when detaching the slide cover 6 from the main body block 4, the connecting claw section 61 is detached from the foreside retaining section 481 forward, and is moved forward through the foreside groove section 483. For that purpose, the connecting claw section 61 that had been arranged at the foreside retaining section 481 is disengaged from the foreside retaining section 481 and is allowed to move forward from the communicating section 485 by pushing the lower side spring arm 631 upward (downward in FIG. 41). The slide cover 6 is slid forward in this state, and the connecting claw section 61 is detached from the main body block 4 forward through the foreside groove section 483. Due to this, the slide cover 6 can be detached from the main body block 4 (cutter body 2).

In the present embodiment, in the attached state S, since the slide cover 6 covers the replaceable blade 3 by locking the slide cover 6 at the foreside position CF, user's safety can be ensured, and damage to the replaceable blade 3 can be prevented. Further, when using the cutter 1, smooth usage of the cutter 1 can be ensured by locking the slide cover 6 at the rear-side position CR.

Further, the connecting claw section 61 of the slide cover 6 is slidably arranged in the slide groove section 48, and connected with the foreside retaining section 481 or the rear-side retaining section 482 so that the slide cover is locked at the foreside position CF or the rear-side position CR. Due to this, the slide cover 6 can easily be moved and locked between the foreside position CF and the rear-side position CR.

Further, since the slide groove section 48 is formed at the back face of the main body block 4, the slide groove section 48 is to be opened to an opposite side of an opened side of the container section 45, thus, freedom degree of arrangement thereof can be improved.

Further, the slide groove section 48 includes the foreside groove section 483 and the rear-side groove section 484, and the communicating section 485 between the foreside retaining section 481 and the foreside groove section 483 and the communicating section 486 between the foreside retaining section 481 and the rear-side groove section 484 are formed on opposite sides of one another in the height direction Z. Due to this, the slide cover 6 can easily be attached and detached with respect to the cutter body 2, and can surely be fixed at the foreside position CF. That is, with the foreside groove section 483 being provided, the slide cover 6 can easily be attached and detached with respect to the cutter body 2 along the longitudinal direction Y thereof while passing the connecting claw section 61 through the foreside groove section 483. Further, by having the communicating section 485 between the foreside retaining section 481 and the foreside groove section 483 and the communicating section 486 between the foreside retaining section 481 and the rear-side groove section 484 formed on the opposite sides of one another in the height direction Z, it is made easier for the connecting claw section 61 to easily and surely be arranged at the foreside retaining section 481. For example, when sliding the slide cover 6 from the rear-side position CR to the foreside position CF to arrange the connecting claw section 61 at the foreside retaining section 481, the connecting claw section 61 is prevented from passing though the foreside retaining section 481 by momentum thereof.

Further, the slide cover 6 includes the pair of spring arms 631, and the pair of spring arms 631 are coupled by the coupling section 632 to one another at the fore end sections, and the connecting claw section 61 is formed at the coupling section 632. Due to this, the connecting claw section 61 can easily be displaced in the height direction Z by the elastic deformation of the spring arms 631.

Further, the slide cover 6 is provided with the front side pressing section 64. Due to this, when the slide cover 6 is arranged at the rear-side position CR, the flexible section 53 of the slide block 5 can be pushed in the thickness direction X via the front side pressing section 64. Due to this, the work to release the attached state S by bending the slide block 5 can easily be performed.

In addition, similar advantageous effect as that of the first embodiment is obtained.

In the cutter 1 of the present embodiment, a front side opening section (not shown in the drawing) may be provided in the slide cover 6 instead of the front side pressing section 64 provided in the slide cover 6. That is, the slide cover 6 may be provided with a front side opening section 64 that exposes the flexible section 53 at a position opposite the flexible section 53 in the slide block 5 when it is arranged at the rear-side position CR. In this case, the flexible section 53 of the slide block 5 exposed from the front side opening section 64 can be pushed in the thickness direction X when the slide cover 6 is arranged at the rear-side position CR. Due to this also, the work to release the attached state S by bending the slide block 5 can easily be performed.

### (Third Embodiment)

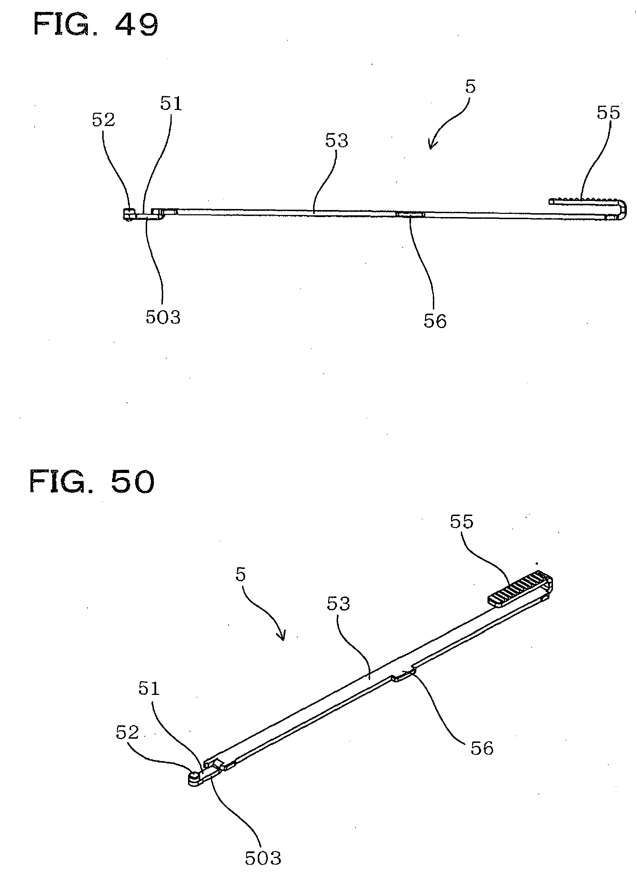

As shown in FIG. 42 to FIG. 50, the present embodiment is relevant to a cutter in which the main body block 4 has a substantially cylindrical shape.

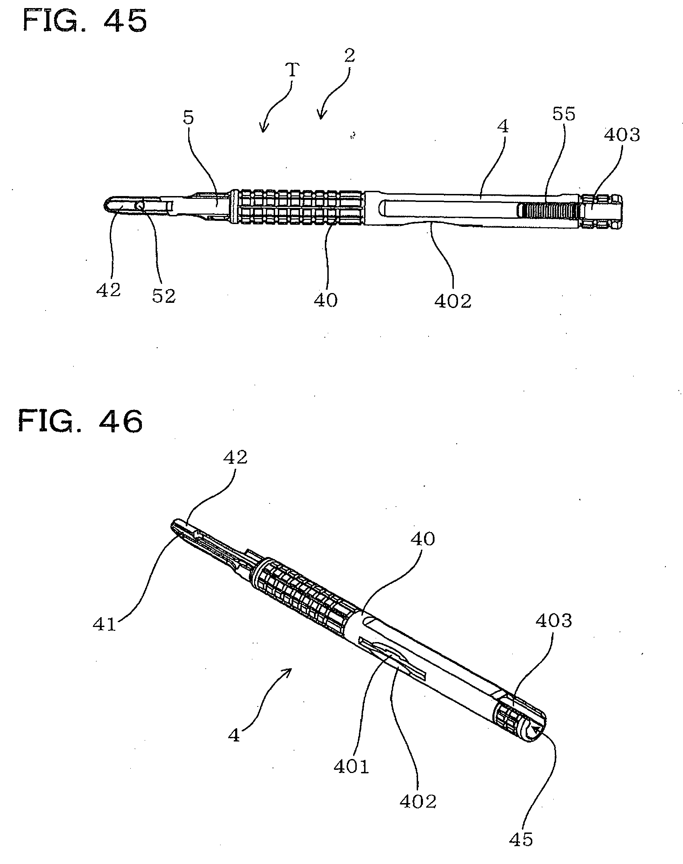

As shown in FIG. 46, portions of the main body block 4 other than the portion in a vicinity of a fore end section at which the main body supporting section 41 and the main body connecting section 42 are provided have the substantially cylindrical shape with an axis of the longitudinal direction Y. Further, this cylinder section 40 having the substantially cylindrical shape penetrates in the longitudinal direction Y, where its inner space is formed as the container section 45 that houses the slide block 5.

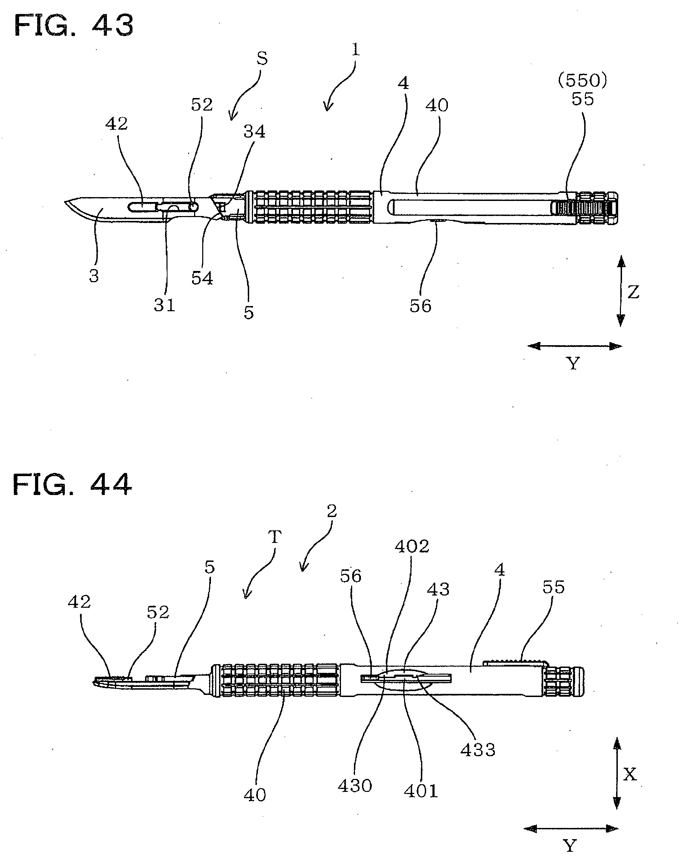

Further, at a lower side in the height direction Z, the cylinder section 40 includes a lower side opening section 401 that is formed along the longitudinal direction Y. As shown in FIG. 42 and FIG. 44, the slide block 5 arranged in the container section 45 has the wing section 56 provided at the lower side opening section 401. The wing section 56 protrudes outward from the lower side opening section 401.

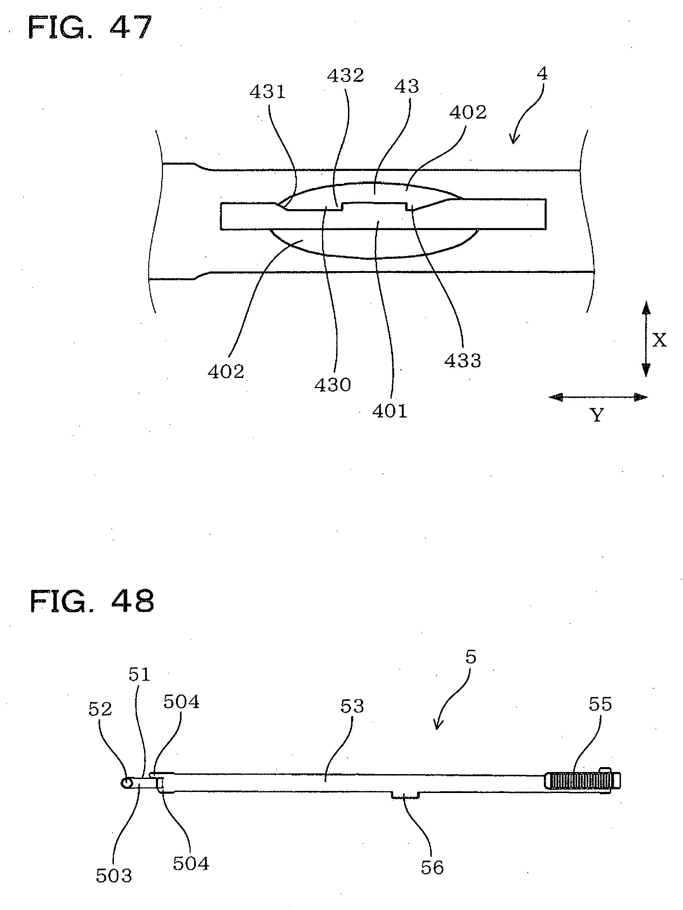

Further, as shown in FIG. 47, the front side retaining section 43 is formed at a front side in a thickness direction X at the lower side opening section 401, and the protruding engaging section 430 protrudes toward a backside therefrom. Further, in the present embodiment, a rear-side engaging section 433 that protrudes toward the backside from the front side retaining section 43 is similarly formed at a rear-side of the protruding engaging section 430.

Further, a lower side concaved section 402 that is concaved toward inside and has a curved surface is formed around the lower side opening section 401.

Further, as shown in FIG. 46, a rear end notch section 403 that is cut out at the front side is formed at a rear end section of the cylinder section 40. The operating section 55 provided at a rear end of the slide block 5 is arranged in the rear end notch section 403.

As shown in FIG. 48 to FIG. 50, the slide block 5 is configured by forming the wing section 56 to protrude toward the lower side in the height direction Z, and does not include the wing section protruding upward as in the first embodiment. Further, the operating section 55 folded toward the front side is formed at the rear end of the slide block 5. Other configurations of the slide block 5 are similar to those in the first embodiment.

In the attached state S in which the replaceable blade 3 is attached to the cutter body 2, a back face of the center part 503 in the vicinity of the fore end section of the slide block 5 and a back face of the operating section 55 at the rear end section abut on the main body block 4, and a space is formed with the main body block 4 at an intermediate portion thereof. The operating section 55 makes contact with a front side face of the cylinder section 40 of the main body block 4.

As shown in FIG. 44 and FIG. 45, in the blade replacing state T, the slide block 5 is in a state of having been moved forward with respect to the main body block 4, and the wing section 56 of the slide block 5 is positioned at a fore end section of the lower side opening section 401. In this state, the replaceable blade 3 is temporarily engaged with the cutter body 2 by causing the main body connecting section 42 of the main body block 4 and the slide connecting section 52 of the slide block 5 to penetrate through the opening section 31 of the replaceable blade 3.

Then, the slide block 5 is retracted rearward with respect to the main body block 4. At this occasion, as shown in FIG. 42 and FIG. 44, the wing section 56 of the slide block 5 slides from the fore end section 431 of the protruding engaging section 430 of the main body block 4 on a back face thereof toward the rear-side. That is, the wing section 56 moves on the back face of the protruding engaging section 430 along the tapered section provided in the fore end section 431 of the protruding engaging section 430. In accompanying this, the slide block 5 bends protrudingly toward the back face side at the flexible section 53.

The slide connecting section 52 pulls the replaceable blade 3 rearward at a rear end of the rear end side opening section 33 by retracting the slide block 5 as described above. Due to this, the replaceable blade 3 is attached to the cutter body 2 as shown in FIG. 42 and FIG. 43.

Further, the wing section 56 of the slide block 5 slides toward the rear-side on the back face of the protruding engaging section 430 and reaches the rear end section 432 of the protruding engaging section 430. While the wing section 56 is sliding on the back face of the protruding engaging section 430, at least the flexible section 53 of the slide block 5 is elastically deformed so as to bend protrudingly toward the backside. Thus, when the wing section 56 of the slide block 5 reaches the rear end section 432 of the protruding engaging section 430, the wing section 56 naturally moves toward the front side by its restoring force, and is engaged at the rear end section 432 of the protruding engaging section 430.

Further, the wing section 56 is brought into a state of being retained from the front side by the front side retaining section 43 at the rear-side of the protruding engaging section 430.

Accordingly, the attached state S in which the replaceable blade 3 is attached to the cutter body 2 is realized (as shown in FIG. 42 and FIG. 43).

In this attached state S, the slide block 5 is not in a state of particularly being bent.

Further, when detaching the replaceable blade 3 from the cutter body 2, the slide block 5 is moved forward with respect to the main body block 4 from the attached state S. At this occasion, the slide block 5 is caused to bend by pushing it toward the backside at the flexible section 53. That is, the wing section 56 protruding from the lower side opening section 401 at the lower face of the main body block 4 is pushed toward the back face side. Specifically, for example, the wing section 56 is pushed toward the backside by a finger or a nail of a user. In accompanying this, the slide block 5 is bent at the flexible section 53. Then, the wing section 56 is displaced toward the backside farther than the protruding engaging section 430 of the main body block 4, and a connected state of the wing section 56 and the protruding engaging section 430 is released. In this state, the wing section 56 moves forward while sliding on the back face of the protruding engaging section 430 by pushing the slide block 5 toward the foreside with respect to the main body block 4.

Further, when moving the slide block 5 forward, the replaceable blade 3 is moved toward the foreside by a rear-side opposing face 54 provided in the slide block 5, which pushes the rear end 34 of the replaceable blade 3. Due to this, the main body connecting section 42 of the main body block 4 relatively moves rearward inside the opening section 31 of the replaceable blade 3, and moves from the fore end side opening section 32 to the rear end side opening section 33. Then, when the slide block 5 is moved forward until the slide connecting section 52 abuts on the main body connecting section 42, both the main body connecting section 42 and the slide connecting section 52 are arranged in the rear end side opening section 33. In this state, the replaceable blade 3 can be detached to the front side.

Other configurations are similar to those of the first embodiment.

In the present embodiment also, similar advantageous effects as those of the first embodiment may be obtained.

## Claims

1. A cutter (1) configured by detachably attaching a replaceable blade (3) to a cutter body (2), wherein
the cutter body (2) is configured of a main body block (4) and a slide block (5) retained with respect to the main body block (4) so as to be able to move in a longitudinal direction (Y) of the cutter (1),
the main body block (4) includes a container section (45) that houses the slide block (5) so as to allow the slide block (5) to move in the longitudinal direction (Y), a main body supporting section (41) that supports a back face of the replaceable blade (3), and a main body connecting section (42) that connects with the replaceable blade (3) from a front face side,
the slide block (5) includes a rear-side supporting section (51) that supports the back face of the replaceable blade (3) at a rear side of the main body supporting section (41), a slide connecting section (52) that connects with the replaceable blade (3) so as to retract the replaceable blade (3) toward the rear side, **characterized by** a flexible section (53) that bends in a thickness direction (X) of the replaceable blade (3) at the rear side of the rear-side supporting section (51), and a wing section (56) that protrudes from a part of the flexible section (53) toward at least one side in a height direction (Z) perpendicular to the longitudinal direction (Y) and the thickness direction (X),
a front side retaining section (43) that retains the wing section (56) from a front side is formed in at least one side of the container section (45) in the height direction (Z), and the front side retaining section (43) includes a protruding engaging section (430) that protrudes toward a backside, and
in an attached state (S) in which the replaceable blade (3) is attached to the cutter body (2), the wing section (56) is engaged from a foreside by the protruding engaging section (430), and the wing section (56) is configured capable of being released from the protruding engaging section (430) by bending the flexible section (53) of the slide block (5) from the attached state (S).

2. The cutter (1) according to claim 1, wherein the protruding engaging section (430) includes a tapered section at a fore end section (431), and a protruding amount of the tapered section decreases toward a fore end side.

3. The cutter (1) according to claim 1 or 2, wherein the replaceable blade (3) has flexibility in the thickness direction (X), and elastically deforms in the attached state (S) so as to curve in the thickness direction (X) protrudingly toward a backside.

4. The cutter (1) according to claim 3, wherein in the attached state (S), the rear-side supporting section (51) protrudes toward a front side in the thickness direction (X) farther than the main body supporting section (41).

5. The cutter (1) according to any one of claims 1 to 4, wherein the slide block (5) includes a rear-side opposing face (54) that opposes a rear end (34) of the replaceable blade (3) in the longitudinal direction (Y), and
the backside opposing face is configured to press the replaceable blade (3) toward the foreside by the backside opposing face abutting on the back end of the replaceable blade (3) when the slide block (5) is moved forward with respect to the main body block (4).

6. The cutter (1) according to any one of claims 1 to 5,
wherein the wing section (56) is formed protruding in both sides in the height direction (Z) of the slide block (5), the front side retaining section (43) is formed at each of both sides in the height direction (Z) of the container concave section, and each of the front side retaining sections (43) at the both sides includes the protruding engaging section (430).

7. The cutter (1) according to any one of claims 1 to 6, further comprising a cylindrical slide cover (6) that is attached slidably in the longitudinal direction (Y) with respect to the main body block (4) and opens in both sides along the longitudinal direction (Y),
wherein the slide cover (6) is configured capable of being locked at a foreside position (CF) where the replaceable blade (3) is covered and a rear-side position (CR) where the replaceable blade (3) is exposed.

8. The cutter (1) according to claim 7, wherein
the slide cover (6) includes a connecting claw section (61) formed toward inside in the thickness direction (X),
the connecting claw section (61) is configured capable of being elastically displaced in the height direction (Z) with respect to a cover main body (60) of the slide cover (6),
the main body block (4) includes a slide groove section (48) formed in the longitudinal direction (Y),
a foreside retaining section (481) and a rear-side retaining section (482) that respectively retain the connecting claw section (61) from fore and rear directions are formed at a foreside and a rear-side of the slide groove section (48), and
the connecting claw section (61) is slidably arranged in the slide groove section (48), and connected with the foreside retaining section (481) or the rear-side retaining section (482) so that the slide cover (6) is locked at the foreside position (CF) or the rear-side position (CR).

9. The cutter (1) according to claim 7 or 8, wherein
the slide groove section (48) includes a foreside groove section (483) that extends from the foreside retaining section (481) toward a foreside and opened toward the foreside of the main body block (5), and a rear-side groove section (484) that extends from the foreside retaining section (481) toward the rear side and is coupled to the rear-side retaining section (484), and
a communicating section (485) for connection between the foreside retaining section (481) and the foreside groove section (483) is formed at opposite sides of a communicating section (486) for connection between the foreside retaining section (481) and the rear-side groove section (484) in the height direction (Z).

10. The cutter (1) according to any one of claims 7 to 9,
wherein the slide cover (6) includes a pair of spring arms (631) capable of elastically deforming in the height direction (Z) at both sides in the height direction (Z), and the pair of spring arms (631) extends from a continuing section from the cover main body (60) toward the foreside and is coupled by a coupling section (632) to one another at fore end sections, and
the connecting claw section (61) is formed in the coupling section (632).

11. The cutter (1) according to any one of claims 6 to 10, wherein the slide cover (6) is configured by including a front side pressing section (64) capable of elastically deforming in the thickness direction (X) at a position opposite the flexible section (53) of the slide block (5) when the slide cover (6) is arranged at the rear-side position (CR).

12. The cutter according to any one of claims 6 to 10, wherein the slide cover (6) is configured by including a front side opening section (64) that exposes the flexible section (53) at the position that opposes the flexible section (53) of the slide block (5) when the slide cover (6) is arranged at the rear-side position (CR).

## Patentansprüche

1. Schneideinrichtung (1), die durch lösbares Befestigen einer austauschbaren Klinge (3) an einem Schneideinrichtungskörper (2) ausgebildet ist, bei der
der Schneideinrichtungskörper (2) aus einem Hauptkörperbauteil (4) und einem Schiebebauteil (5), das bezüglich des Hauptkörperbauteils (4) gehalten wird, sodass es in einer Längsrichtung (Y) der Schneideinrichtung (1) bewegbar ist, ausgebildet ist,
das Hauptkörperbauteil (4) einen Behälterabschnitt (45), der das Schiebebauteil (5) so aufnimmt, dass dem Schiebebauteil (5) ermöglicht wird, sich in der Längsrichtung (Y) zu bewegen, einen Hauptkörpertrageabschnitt (41), der eine Rückfläche der austauschbaren Klinge (3) trägt, und einen Hauptkörperverbindungsabschnitt (42), der mit der austauschbaren Klinge (3) von einer Vorderflächenseite verbunden ist, aufweist,
das Schiebebauteil (5) einen Rückseitentrageabschnitt (51), der die Rückfläche der austauschbaren Klinge (3) an einer Rückseite des Hauptkörpertrageabschnitts (41) trägt, einen Schiebeverbindungsabschnitt (62), der mit der austauschbaren Klinge (3) zum Einziehen der austauschbaren Klinge (3) in Richtung zu der Rückseite verbunden ist, aufweist, **gekennzeichnet durch** einen flexiblen Abschnitt (53), der sich in einer Dickenrichtung (X) der austauschbaren Klinge (3) an der Rückseite des Rückseitentrageabschnitts (51) biegt, und einen Flügelabschnitt (56), der von einem Teil des flexiblen Abschnitts (53) in Richtung zu zumindest einer Seite in einer Höhenrichtung (Z), die senkrecht zu der Längsrichtung (Y) und der Dickenrichtung (X) ist, vorsteht,
ein Vorderseitenhalteabschnitt (43), der den Flügelabschnitt (56) von einer Vorderseite hält, in zumindest einer Seite des Behälterabschnitts (45) in der Höhenrichtung (Z) ausgebildet ist, und der Vorderseitenhalteabschnitt (43) einen vorstehenden Eingriffsabschnitt (430), der in Richtung zu einer Rückseite vorsteht, aufweist, und
in einem Befestigungszustand (S), in dem die austauschbare Klinge (3) an dem Schneideinrichtungskörper (2) befestigt ist, der Flügelabschnitt (56) von einer Vorderseite durch den vorstehenden Eingriffsabschnitt (430) in Eingriff ist, und der Flügelabschnitt (56) dazu ausgebildet ist, in der Lage zu sein, von dem vorstehenden Eingriffsabschnitt (430) **durch** Biegen des flexiblen Abschnitts (53) des Schiebebauteils (5) aus dem Befestigungszustand (S) gelöst zu werden.

2. Schneideinrichtung (1) nach Anspruch 1, bei der der vorstehende Eingriffsabschnitt (430) einen sich verjüngenden Abschnitt an einem Vorderendabschnitt (431) aufweist, und ein Vorstehmaß des sich verjüngenden Abschnitts sich in Richtung zu einer Vorderendseite verringert.

3. Schneideinrichtung (1) nach Anspruch 1 oder 2, bei der die austauschbare Klinge (3) in der Dickenrichtung (X) flexibel ist und sich in dem Befestigungszustand (S) so elastisch verformt, dass sie in der Dickenrichtung (X) in Richtung zu einer Rückseite gekrümmt vorsteht.

4. Schneideinrichtung (1) nach Anspruch 3, bei der in dem Befestigungszustand (S) der Rückseitentrageabschnitt (51) in Richtung zu einer Vorderseite in der Dickenrichtung (X) weiter als der Hauptkörpertrageabschnitt (41) vorsteht.

5. Schneideinrichtung (1) nach einem der Ansprüche 1 bis 4, bei der das Schiebebauteil (5) eine der Rückseite gegenüberliegende Fläche (54), die einem Hinterende (34) der austauschbaren Klinge (3) in der Längsrichtung (Y) gegenüberliegt, aufweist, und
die der Rückseite gegenüberliegende Fläche dazu ausgebildet ist, die austauschbare Klinge (3) in Richtung zu der Vorderseite durch die der Rückseite gegenüberliegende Fläche, die an das Hinterende der austauschbaren Klinge (3) anstößt, wenn das Schiebebauteil (5) bezüglich des Hauptkörperbauteils (4) nach vorne bewegt wird, zu drücken.

6. Schneideinrichtung (1) nach einem der Ansprüche 1 bis 5, bei der
der Flügelabschnitt (56) zu beiden Seiten in der Höhenrichtung (Z) des Schiebebauteils (5) vorstehend ausgebildet ist, der Vorderseitenhalteabschnitt (43) an jeder der beiden Seiten in der Höhenrichtung (Z) des konkaven Behälterabschnitts ausgebildet ist, und jede der Vorderseitenhalteabschnitte (43) an den beiden Seiten den vorstehenden Eingriffsabschnitt (430) aufweist.

7. Schneideinrichtung (1) nach einem der Ansprüche 1 bis 6, ferner aufweisend eine zylindrische Schiebeabdeckung (6), die bezüglich des Hauptkörperbauteils (4) in der Längsrichtung (Y) verschiebbar befestigt ist und an beiden Seiten entlang der Längsrichtung (Y) geöffnet ist,
wobei die Schiebeabdeckung (6) dazu ausgebildet ist, dazu in der Lage zu sein, in einer Vorderseitenposition (CF), in der die austauschbare Klinge (3) abgedeckt ist, und einer Rückseitenposition (CR), in der die austauschbare Klinge (3) freiliegt, gesichert zu sein.

8. Schneideinrichtung (1) nach Anspruch 7, bei der
die Schiebeabdeckung (6) einen Verbindungsklauenabschnitt (61) aufweist, der in Richtung zu einem Inneren in der Dickenrichtung (X) ausgebildet ist,
der Verbindungsklauenabschnitt (61) dazu ausgebildet ist, dazu in der Lage zu sein, in der Höhenrichtung (Z) bezüglich eines Abdeckungshauptkörpers (60) der Schiebeabdeckung (6) elastisch versetzt zu sein,
das Hauptkörperbauteil (4) einen Schiebenutabschnitt (48) aufweist, der in der Längsrichtung (Y) ausgebildet ist,
ein Vorderseitenhalteabschnitt (481) und eine Rückseitenhalteabschnitt (482), die jeweils den Verbindungsklauenabschnitt (61) von einer Vorder- bzw. einer Rückrichtung halten, an einer Vorderseite bzw. einer Rückseite des Schiebenutabschnitts (48) ausgebildet sind, und
der Verbindungsklauenabschnitt (61) verschiebbar in dem Schiebenutabschnitt (48) angeordnet ist und mit dem Vorderseitenhalteabschnitt (481) oder dem Rückseitenhalteabschnitt (482) so verbunden ist, dass die Schiebeabdeckung (6) an der Vorderseitenposition (CF) oder der Rückseitenposition (CR) gesichert ist.

9. Schneideinrichtung (1) nach Anspruch 7 oder 8, bei der
der Schiebenutabschnitt (48) einen Vorderseitennutabschnitt (483), der sich von dem Vorderseitenhalteabschnitt (481) in Richtung zu einer Vorderseite erstreckt und in Richtung zu der Vorderseite des Hauptkörperbauteils (5) geöffnet ist, und einen Rückseitennutabschnitt (484), der sich von dem Vorderseitenhalteabschnitt (481) in Richtung zu der Rückseite erstreckt und mit dem Rückseitenhalteabschnitt (484) verbunden ist, aufweist, und
ein Verbindungsabschnitt (485) zum Verbinden zwischen dem Vorderseitenhalteabschnitt (481) und dem Vorderseitennutabschnitt (483) an gegenüberliegenden Seiten eines Verbindungsabschnitts (486) zum Verbinden zwischen dem Vorderseitenhalteabschnitt (481) und dem Rückseitennutabschnitt (484) in der Höhenrichtung (Z) ausgebildet ist.

10. Schneideinrichtung (1) nach einem der Ansprüche 7 bis 9, bei der
die Schiebeabdeckung (6) ein Paar Federarme (631), die in der Lage sind, sich in der Höhenrichtung (Z) elastisch zu verformen, an beiden Seiten in der Höhenrichtung (Z) aufweist, und das Paar Federarme (631) sich von einem Anschlussabschnitt von dem Abdeckungshauptkörper (60) in Richtung zu der Vorderseite erstreckt und miteinander durch einen Verbindungsabschnitt (632) an Vorderendabschnitten verbunden ist, und
der Verbindungsklauenabschnitt (61) in dem Verbindungsabschnitt (632) ausgebildet ist.

11. Schneideinrichtung (1) nach einem der Ansprüche 6 bis 10, bei der die Schiebeabdeckung (6) durch Aufweisen eines Vorderseitendrückabschnitts (64), der in der Lage ist, sich in der Dickenrichtung (X) an einer Position gegenüber dem flexiblen Abschnitt (53) des Schiebebauteils (5), wenn die Schiebeabdeckung (6) in der Rückseitenposition (CR) angeordnet ist, elastisch zu verformen, ausgebildet ist.

12. Schneideinrichtung nach einem der Ansprüche 6 bis 10, bei der die Schiebeabdeckung (6) durch Aufweisen eines Vorderseitenöffnungsabschnitts (64), der den flexiblen Abschnitt (53) an der Position, die dem flexiblen Abschnitt (53) des Schiebebauteils (5) gegenüberliegt, wenn die Schiebeabdeckung (6) in der Rückseitenposition (CR) angeordnet ist, freilegt, ausgebildet ist.

## Revendications

1. Couteau (1) configuré en attachant de manière détachable une lame remplaçable (3) à un corps de couteau (2), dans lequel
le corps de couteau (2) est configuré avec un bloc de corps principal (4) et un bloc coulissant (5) retenu par rapport au bloc de corps principal (4) de sorte à être capable de se déplacer dans une direction longitudinale (Y) du couteau (1),
le bloc de corps principal (4) inclut une section de contenant (45) qui loge le bloc coulissant (5) de sorte à permettre au bloc coulissant (5) de se déplacer dans la direction longitudinale (Y), une section de support de corps principal (41) qui supporte une face arrière de la lame remplaçable (3), et une section de liaison de corps principal (42) qui est reliée à la lame remplaçable (3) depuis un côté de face avant,
le bloc coulissant (5) inclut une section de support de côté arrière (51) qui supporte la face arrière de la lame remplaçable (3) sur un côté arrière de la section de support de corps principal (41), une section de liaison coulissante (52) qui est reliée à la lame remplaçable (3) de sorte à rétracter la lame remplaçable (3) vers le côté arrière, **caractérisé par** une section flexible (53) qui plie dans une direction de l'épaisseur (X) de la lame remplaçable (3) sur le côté arrière de la section de support de côté arrière (51) et une section d'aile (56) qui fait saillie d'une partie de la section flexible (53) vers au moins un côté dans une direction de la hauteur (Z) perpendiculaire à la direction longitudinale (Y) et la direction de l'épaisseur (X),
une section de retenue de côté avant (43) qui retient la section d'aile (56) depuis un côté avant est formée dans au moins un côté de la section de contenant (45) dans la direction de la hauteur (Z), et la section de retenue de côté avant (43) inclut une section de mise en prise en saillie (430) qui fait saillie vers un côté arrière, et
dans un état attaché (S) dans lequel la lame remplaçable (3) est attachée au corps de couteau (2), la section d'aile (56) est mise en prise depuis un côté avant par la section de mise en prise en saillie (430), et la section d'aile (56) est configurée de manière à être capable d'être libérée de la section de mise en prise en saillie (430) par pliage de la section flexible (53) du bloc coulissant (5) depuis l'état attaché (S).

2. Couteau (1) selon la revendication 1, dans lequel la section de mise en prise en saillie (430) inclut une section effilée sur une section d'extrémité avant (431), et une quantité en saillie de la section effilée diminue vers un côté d'extrémité avant.

3. Couteau (1) selon la revendication 1 ou 2, dans lequel la lame remplaçable (3) présente une flexibilité dans la direction de l'épaisseur (X) et se déforme élastiquement dans l'état attaché (S) de sorte à se courber dans la direction de l'épaisseur (X) en saillie vers un côté arrière.

4. Couteau (1) selon la revendication 3, dans lequel dans l'état attaché (S), la section de support de côté arrière (51) fait saillie vers un côté avant dans la direction de l'épaisseur (X) plus loin que la section de support de corps principal (41).

5. Couteau (1) selon l'une quelconque des revendications 1 à 4, dans lequel le bloc coulissant (5) inclut une face en regard du côté arrière (54) qui est en regard d'une extrémité arrière (34) de la lame remplaçable (3) dans la direction longitudinale (Y) et
la face en regard du côté arrière est configurée pour presser la lame remplaçable (3) vers le côté avant par la face en regard du côté arrière butant contre l'extrémité arrière de la lame remplaçable (3) lorsque le bloc coulissant (5) est déplacé vers l'avant par rapport au bloc de corps principal (4).

6. Couteau (1) selon l'une quelconque des revendications 1 à 5, dans lequel
la section d'aile (56) est formée en saillie dans les deux côtés dans la direction de la hauteur (Z) du bloc coulissant (5), la section de retenue de côté avant (43) est formée sur chacun des deux côtés dans la direction de la hauteur (Z) de la section concave de contenant et chacune des sections de retenue côté avant (43) sur les deux côtés inclut la section de mise en prise en saillie (430).

7. Couteau (1) selon l'une quelconque des revendications 1 à 6, comprenant en outre un couvercle coulissant cylindrique (6) qui est attaché de manière coulissante dans la direction longitudinale (Y) par rapport au bloc de corps principal (4) et s'ouvre dans deux côtés le long de la direction longitudinale (Y),
dans lequel le couvercle coulissant (6) est configuré de manière à être capable d'être verrouillé dans une position côté avant (CF) où la lame remplaçable (3) est couverte et une position côté arrière (CR) où la lame remplaçable (3) est exposée.

8. Couteau (1) selon la revendication 7, dans lequel le couvercle coulissant (6) inclut une section de griffe de liaison (61) formée vers l'intérieur dans la direction de l'épaisseur (X),
la section de griffe de liaison (61) est configurée de manière à être capable d'être déplacée élastiquement dans la direction de la hauteur (Z) par rapport à un corps principal de couvercle (60) du couvercle coulissant (6),
le bloc de corps principal (4) inclut une section de rainure coulissante (48) formée dans la direction longitudinale (Y),
une section de retenue de côté avant (481) et une section de retenue de côté arrière (482) qui retiennent respectivement la section de griffe de liaison (61) depuis les directions avant et arrière sont formées sur un côté avant et un côté arrière de la section de rainure coulissante (48) et
la section de griffe de liaison (61) est agencée de manière coulissante dans la section de rainure coulissante (48) et reliée à la section de retenue de côté avant (481) ou la section de retenue de côté arrière (482) de sorte que le couvercle coulissant (6) soit verrouillé dans la position de côté avant (CF) ou la position de côté arrière (CR).

9. Couteau (1) selon la revendication 7 ou 8, dans lequel la section de rainure coulissante (48) inclut une section de rainure de côté avant (483) qui s'étend depuis la section de retenue de côté avant (481) vers un côté avant et ouverte vers le côté avant du bloc de corps principal (5), et une section de rainure de côté arrière (484) qui s'étend depuis la section de retenue de côté avant (481) vers le côté arrière et est couplée à la section de retenue de côté arrière (484) et
une section de communication (485) pour la liaison entre la section de retenue de côté avant (481) et la section de rainure de côté avant (483) est formée sur des côtés en regard d'une section de communication (486) pour la liaison entre la section de retenue de côté avant (481) et la section de rainure de côté arrière (484) dans la direction de la hauteur (Z).

10. Couteau (1) selon l'une quelconque des revendications 7 à 9, dans lequel
le couvercle coulissant (6) inclut une paire de bras à ressort (631) capable de se déformer élastiquement dans la direction de la hauteur (Z) sur les deux côtés dans la direction de la hauteur (Z) et la paire de bras à ressort (631) s'étend depuis une section en continu depuis le corps principal de couvercle (60) vers le côté avant et est couplée par une section de couplage (632) à une autre sur des sections d'extrémité avant et la section de griffe de liaison (61) est formée dans la section de couplage (632).

11. Couteau (1) selon l'une quelconque des revendications 6 à 10, dans lequel le couvercle coulissant (6) est configuré en incluant une section de pressage de côté avant (64) capable de se déformer élastiquement dans la direction de l'épaisseur (X) dans une position en regard de la section flexible (53) du bloc coulissant (5) lorsque le couvercle coulissant (6) est agencé dans la position de côté arrière (CR).

12. Couteau (1) selon l'une quelconque des revendications 6 à 10, dans lequel le couvercle coulissant (6) est configuré en incluant une section d'ouverture de côté avant (64) qui expose la section flexible (53) dans la position qui fait face à la section flexible (53) du bloc coulissant (5) lorsque le couvercle coulissant (6) est agencé dans la position de côté arrière (CR).
